# EUROPÄISCHE PATENTANMELDUNG

(11) **EP 2 581 878 A2**
(43) Veröffentlichungstag der Anmeldung: **17.04.2013**
(21) Anmeldenummer: 12188243.5
(22) Anmeldetag: 11.10.2012
(51) Int. Cl.: G06T 7/00, G06T 7/60, G06K 9/00

(54) **Verfahren und Vorrichtung zur Quantifizierung einer Schädigung eines Hautgewebeschnittes**

(30) Priorität: 11.10.2011 DE 102011084286
(71) Anmelder: Fraunhofer-Gesellschaft zur Förderung der angewandten Forschung e.V., 80686 München (DE)
(72) Erfinder: Münzenmayer, Christian, 91469 Hagenbüchach (DE); Wittenberg, Thomas, 91054 Erlangen (DE); Gryanik, Alexander, 91054 Erlangen (DE); Steckhan, Dirk, 81827 München (DE)
(74) Vertreter: Schenk, Markus

(57) **Zusammenfassung**

Es wird eine Vorrichtung zur Quantifizierung einer Schädigung eines Hautgewebeschnittes anhand eines Farbbildes des Hautgewebeschnittes beschrieben, die eine Einrichtung zum Konvertieren des Farbbildes in ein Grauwertbild; eine Einrichtung zum Zuweisen von Bildpunkten des Grauwertbildes zu einem jeweiligen Label aus Hintergrund, Epidermis oder Dermis unter Verwendung eines Bildverarbeitungsalgorithmus, um ein Labelbild zu erhalten; eine Einrichtung zum, entlang einer Richtung, die von einer der Dermis abgewandten Seite auf die Epidermis des Hautgewebeschnittes weist, Bestimmen eines Außeroberflächenrands der Epidermis und Anwenden eines Füllalgorithmus von dem Außenoberflächenrand aus auf das Labelbild, um einen mit dem Außeroberflächenrand zusammenhängenden und sich von dem Außeroberflächenrand in der Richtung zur Dermis hin erstreckenden Epidermisbereich zu bestimmen; eine Einrichtung zum Lokalisieren der Epidermis in dem Labelbild unter Verwendung des Epidermisbereichs; und eine Einrichtung zum Bestimmen von Parametern, die charakteristische Eigenschaften einer Form der lokalisierten Epidermis beschreiben umfasst.

## Beschreibung

Die vorliegende Erfindung bezieht sich auf die Quantifizierung einer Schädigung eines Hautgewebeschnittes, wie zum Beispiel zur halbautomatischen oder sogar vollautomatischen Quantifizierung einer schädigenden Wirkung einer die Schädigung hervorrufenden Substanz.

Zunehmend werden komplexere, dreidimensionale Zellsysteme entwickelt, die u.a. für die Herstellung menschlicher Gewebe und Organe eingesetzt werden können. Diese sog. Modelle lassen sich nicht nur als Transplantate, sondern gleichermaßen auch als *in vitro* Testsysteme für die Pharma-, Kosmetik- und Chemieindustrie einsetzen. Mit diesen organoiden Modellen können u.a. sicherheitstoxikologische Prüfungen durchgeführt und schädliche Einflüsse von Substanzen auf die Haut untersucht und analysiert werden. Die Auswertungen werden überwiegend mittels histologischer Diagnostik, d.h. durch Herstellung von Gewebeschnitten, die mit einer Hämatoxilin/Eosin-Färbung präpariert werden, durchgeführt. Diese Technik ist sehr aufwändig und zeitintensiv, da sie ausschließlich manuell mittels Sichtproben unter dem Mikroskop und unter Zerstörung von Referenzexemplaren erfolgen.

Um eine effiziente, standardisierte, objektivier- und reproduzierbare Beurteilung von künstlichem Gewebe zerstörungsfrei - und ideal am unfixierten und somit am »nativen« Produkt - zu ermöglichen, fehlt es aktuell an geeigneten Technologien.

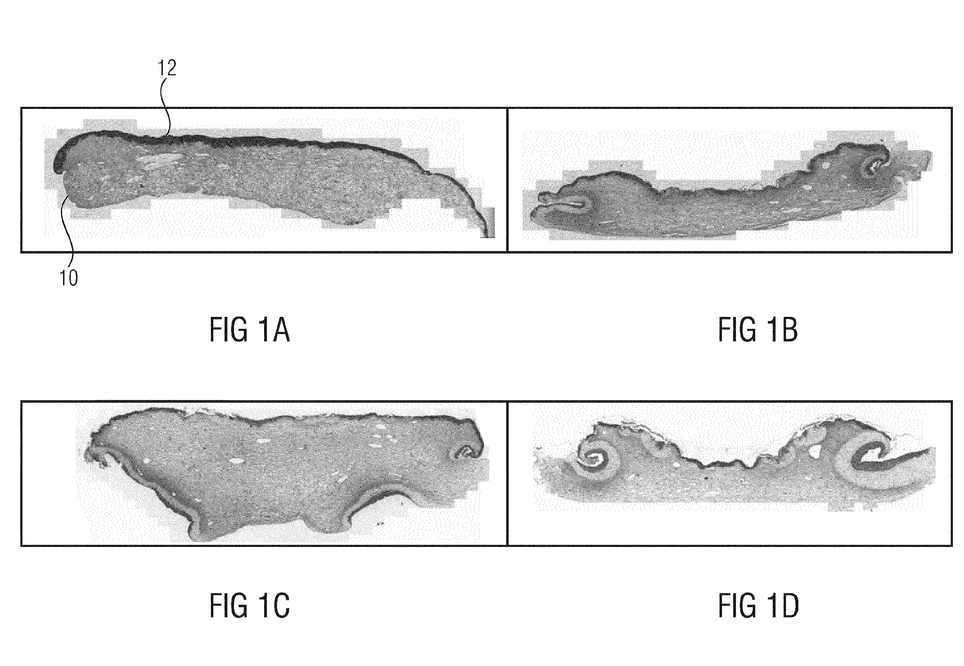

Sicherlich wurden schon Tests von Substanzen im Tierversuch bzw. inzwischen auch mit *in vitro* Testsystemen wie oben beschrieben durchgeführt. Dabei erfolgt eine manuelle Sichtung der Gewebeschnitte von *in vitro* Testsystemen am Mikroskop. Es gibt auch digitale Bildverarbeitungssysteme für die Mikroskopie, wie zum Beispiel zur Klassifikation von Blutzellen oder auch zur Analyse von histologischen Schnitten, wie zum Beispiel von Definiens Cellenger/Tissue Studio, Tissuegnostics TissueFAXS, WestMedica Digital pathology. Allerdings kann mit all diesen Maßnahmen die durch toxische Substanzen an der obersten Hautschicht entstehende Schädigung, nämlich die Erosion, Auflösung oder dergleichen, wie sie exemplarisch in Fig. 1a-1d für unterschiedlich lange Expositionen für eine exemplarische toxische Substanz gezeigt ist, nicht quantifiziert werden.

Fig. 1a zeigt den Hautgewebeschnitt ohne Exposition zu der toxischen Substanz, wobei ersichtlich ist, dass die die Dermis 10 des Hautgewebeschnittes abdeckende Epidermis 12 relativ glatt und stark bezogen auf die Dicke ist. Beide Eigenschaften der Epidermis 12 verschlechtern sich mit zunehmender Expositionszeitdauer (vgl. 1b über Fig. 1c bis zu Fig. 1d). Die Expositionszeiten betrugen 0 Sekunden, 30 Sekunden, 60 Sekunden bzw. 90 Sekunden.

Es wäre wünschenswert, ein Konzept zur Quantifizierung einer Schädigung eines Hautgewebeschnittes an der Hand zu haben, das eine Automatisierung erleichtert, und somit aber auch die Erzielung der damit einhergehenden Vorteile, nämlich der geringere personelle Aufwand und die damit verringerten Kosten, die Reproduzierbarkeit der Quantifizierung usw.

Diese Aufgabe wird durch den Gegenstand der beigefügten unabhängigen Patentansprüche gelöst.

Gemäß einem Ausführungsbeispiel der vorliegenden Erfindung umfasst eine Vorrichtung zur Quantifizierung einer Schädigung einer Hautgewebeschnittes anhand eines Farbbildes des Hautgewebeschnittes eine Einrichtung zum Konvertieren des Farbbildes in ein Grauwertbild; eine Einrichtung zum Zuweisen von Bildpunkten des Grauwertbildes zu einem jeweiligen Label aus Hintergrund, Epidermis oder Dermis unter Verwendung eines Bildverarbeitungsalgorithmus, um ein Labelbild zu erhalten; eine Einrichtung zum, entlang einer Richtung, die von einer der Dermis abgewandten Seite auf die Epidermis des Hautgewebeschnittes weist, Bestimmen eines Außeroberflächenrands der Epidermis und Anwenden eines Füllalgorithmus von dem Außenoberflächenrand aus auf das Labelbild, um einen mit dem Außeroberflächenrand zusammenhängenden und sich von dem Außeroberflächenrand in der Richtung zur Dermis hin erstreckenden Epidermisbereich zu bestimmen; eine Einrichtung zum Lokalisieren der Epidermis in dem Labelbild unter Verwendung des Epidermisbereichs; und eine Einrichtung zum Bestimmen von Parametern, die charakteristische Eigenschaften einer Form der lokalisierten Epidermis beschreiben.

Bevorzugte Ausführungsbeispiele der vorliegenden Erfindung werden nachfolgend Bezug nehmend auf die beiliegenden Zeichnungen näher erläutert. Es zeigen:
- Fig. 1: Bildbeispiele für Hautgewebeschnitte, nämlich (a) unbehandelt, (b) nach 30-, (c) 60-, (d) 90-sekündige Exposition.
- Fig. 2: Phasen eines Verfahrens zur Quantifizierung einer schädigenden Wirkung einer Substanz gemäß einem Ausführungsbeispiel;
- Fig. 3: ein Flussdiagram eines Verfahrens zur Quantifizierung einer schädigenden Wirkung einer Substanz gemäß einem Ausführungsbeispiel;
- Fig. 4: eine schematische Darstellung einer Vorrichtung zur Farbbildgewinnung;
- Fig. 5a: eine mögliche Objektträgeridentifikation
- Fig. 5b: ein exemplarisches Übersichtsbild in geringer Auflösung
- Fig. 5c: ein exemplarisches Ergebnis einer automatischen Lokalisierung und Segmentierung in Form eines Hochauflösungsbildes an der relevanten Objektträgerposition;
- Fig. 5d: ein Einzelbild in hoher Auflösung des relevanten Bereichs;
- Fig. 6a: eine schematische Veranschaulichung des Aneinanderfügens von einzelnen, überlappenden Sichtfeldern mit mäanderförmigem Ablauf des Scans,
- Fig. 6b: eine schematische Veranschaulichung einer globalen Optimierung bei der Bild-zu-Bild-Registrierung für ein Stitching zu einer Gesamtansicht;
- Fig. 6c und 6d: zeigen jeweils eine zusammengefügte Gesamtaufnahme eines Objekts auf einem Objektträger, und zwar in dem Fall von Fig. 6c lediglich auf Basis der mechanisch ermittelten Aufnahmeorte, und im Fall von Fig. 6d unter Verwendung der Auswertung der Ähnlichkeitsanalyse benachbarter Einzelbilder durch globale Optimierung,
- Fig. 7a: ein exemplarisches Bild mit sechs Hautschnitten mit Verstärkung der Farben in den Regionen der Schnitte mittels Subtraktion des geglätteten Blaukanals vom Grünkanal;
- Fig. 7b: das Bild aus Fig. 7a geglättet;
- Fig. 7c: das Bild aus Fig. 7b mit einem Schwellwert binarisiert;
- Fig. 7d: Das Bild aus Fig. 7c mit mittels morphologischer Operatoren geschlossenen Regionen;
- Fig. 8: ein Flussdiagram eines Verfahrens zur Schädigungsquantifizierung gemäß einem Ausführungsbeispiel;
- Fig. 9: ein Beispiel zur Positionierung von Saatpunkten zur Begrenzung der Auswerte-Region;
- Fig. 10: eine grauwerttransformierte Version der Auswerte-Region aus Fig. 9;
- Fig. 11: ein aus Fig. 10 gewonnenes Labelbild;
- Fig. 12: eine aus Fig. 11 gewonnene vorläufige Epidermisregion gemäß einem Ausführungsbeispiel;
- Fig. 13: die aus Fig. 11 gewonnene vorläufige Epidermisregion unter Entfernung der nicht-interessierenden Anteile gemäß einem Ausführungsbeispiel;
- Fig. 14: die aus Fig. 12 gewonnene vorläufige Epidermisregion unter Entfernung von Auswüchsen gemäß einem Ausführgunsbeispiel;
- Fig. 15: die Epidermis, wie sie sich aus der in Fig. 15 gezeigten Epidermisregion und dem Epidermis-Label-Gebiet aus dem Labelbild von Fig. 11 gemäß einem Ausführgunsbeispiel ergibt;
- Fig. 16: Kleiner Ausschnitt aus der äußere Epidermiskante, wobei der Kreis zeigt, wie die Länge bei der Parameterbestimmung für Parameter 2 berechnet wird;
- Fig. 17: eine Darstellung einzelner Parameter für eine Probe, wobei (a) Anzahl der Pixel in einer Spalte. (b) Länge einzelner Segmente über die Gesamtlänge. (c) Anzahl der Überschneidungen einzelner Kantensegmente mit dem Radius, (d) ein Histogramm von Parameter 2 zeigt, gemäß einem Ausführungsbeispiel;
- Fig. 18: ein Beispiel für ein Skelettierungsergebnis der besimmten Epidermis;
- Fig. 19: eine Darstellung der gemittelten Werte der Parameter 1-5 (Fig. 19a-e) für die Gewebeschnitte aus Fig. 1; und
- Fig.20: eine Darstellung der gemittelten Werte der Parameter Länge über Überschneidung (a) bzw. Länge über Anzahl der abgeschnittenen Äste der Skelettierung (b).

Bevor Bezug nehmend auf die Figuren ein konkretes Ausführungsbeispiel der vorliegenden Erfindung beschrieben wird, soll im Folgenden noch einmal der Versuch unternommen werden, das in der Beschreibungseinleitung der vorliegenden Anmeldung bestehende Problem näher darzustellen, um die Vorteile, die aus den Ausführungsbeispielen der vorliegenden Erfindung resultieren, dem Leser besser verständlich machen zu können.

Insbesondere wurde in der Beschreibungseinleitung der vorliegenden Anmeldung ja schon beschrieben, dass derzeit Hautgewebeschnitte existieren, die künstlich herstellbar sind und somit die Reproduzierbarkeit von Messergebnissen bezüglich einer Quantifizierung von Schädigungen derselben fördern. Beispielsweise ist es dem Fraunhofer-Institut für Grenzflächen- und Bioverfahrenstechnik IGB gelungen, solche Hautmodelle zu erstellen. Sie können mit Testsubstanzen manipuliert werden. Aus solchen manipulierten Proben können dann histologische Schnitte, d.h. Hautgewebeschnitte angefertigt, gefärbt und präpariert werden, um dann analysiert zu werden. Die Analyse ist allerdings derzeit nur manuell durchgeführt worden, was dem Ziel eines geringen Aufwandes in der Reproduzierbarkeit entgegensteht.

Das sog. Tissue Engineering als Basis für die Herstellung von Gewebetransplantaten und zukünftig auch künstlichen Organen beruht auf der Herstellung von bioartifiziellen Konstrukten, die aus vitalen Zellen bzw. einer Zellmatrix und biokompatiblen Trägermaterialien bestehen. Bereits während der Kultivierung von Tissue Engineering Produkten ist eine kontinuierliche Qualitätskontrolle ein essentieller Prozess. Voraussetzung für die Funktionalität eines Transplantates bzw. eines künstlichen Organs ist der Nachweis der physiologischen Strukturierung der Zellen innerhalb des zugehörigen *in vitro* Modells. Speziell im Rahmen der Entwicklung und Prüfung von Arzneimitteln und Kosmetika wurden in den letzten Jahren zunehmend *in vitro* Testsysteme erfolgreich eingesetzt [1; 2]. Entsprechend steigt der Bedarf und die Notwendigkeit an geeigneten objektiven Auswertemethoden für solche Modelle.

Mit den Methoden der modernen Biologie werden aktuell *neue* zell-basierte Testsysteme für die pharmazeutische sowie die Kosmetikindustrie entwickelt, mit denen es möglich sein wird, verschiedene Einflüsse von applizierten Substanzen auf die betroffenen Zellen möglichst unverfälscht, und objektiv zu analysieren. Derart applizierte Substanzen können zu Veränderungen der zellulären Eigenschaften führen, die sich dort bereits frühzeitig zeigen. Solche Mikroveränderungen der künstlichen Gewebe sind wichtige, direkte Parameter für die anschließende Bewertung und Beurteilung der Substanzen. Die Einteilungen des sog. »Irritativen Potenzials« erfolgt im Wesentlichen über histologische Auswertungen, die sehr zeitaufwendig, nicht quantifizierbar und schwer reproduzierbar sind. Solche histologische Verfahren können zudem den physiologischen Aufbau einzelner Proteinvernetzungen zerstören und so den Gewebeaufbau nicht mehr »nativ« wiedergeben. Deshalb ist es schwer möglich die ursprüngliche Form des Gewebeaufbaus strukturell nachzuweisen und Störungen, wie sie durch Substanzapplikation im Aufbau auftreten zu verifizieren. Auf der Grundlage einer *quantitativen* und *objektiven* Histologie könnten diese Falschinterpretationen im Gewebeaufbau vermieden werden und zu einer schnelleren Analyse führen.

Zur Unterstützung einer quantitativen und objektiven histologischen Untersuchung eignen sich besonders bildgebende optische Verfahren, die in den letzten Jahren in der Biomedizin beeindruckende Fortschritte erreicht haben. Insbesondere die Mikroskopie erscheint für eine automatische Auswertung von präparierten Hautmodellen geeignet zu sein.

Deshalb ist es Ziel der nachfolgend beschriebenen Ausführungsbeispiele, ein Konzept für eine automatische, bildbasierte Beschreibung und Quantifizierung von präparierten Hautmodellen im Kontext der Analyse von toxologischen, kosmetischen oder ähnlichen Einflüssen auf das Hautgewebe zu schaffen.

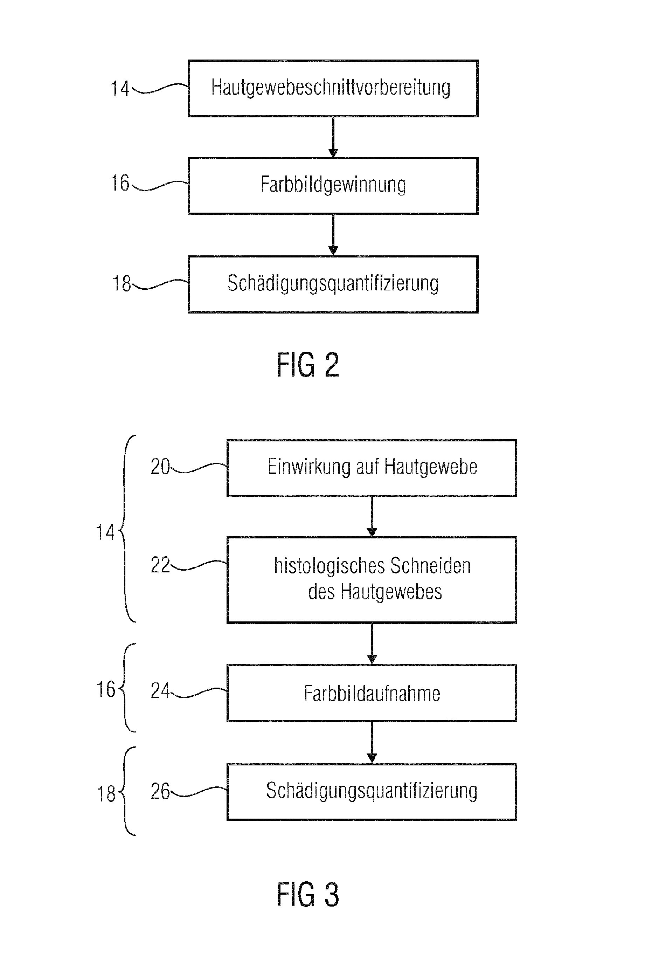

Die nachfolgend beschriebenen Ausführungsbeispiele folgen dementsprechend grundsätzlich dem in Fig. 2 veranschaulichten dreiphasigen Konzept, wonach in einer ersten Phase 14 zunächst ein oder mehrere Hautgewebeschnitte vorbereitet werden, in einer zweiten Phase 16 ein oder mehrere Farbbilder des oder der jeweiligen Hautgewebeschnitte gewonnen werden, und in einer dritten Phase 18 die Farbbildauswertung mit der Schädigungsquantifizierung erfolgt. Zur Vermeidung zu großen Informationsverlustes zwischen den Phasen 14 und 16 sollte gewährleistet werden, dass bei der Gewinnung des jeweiligen Farbbildes auf eine ausreichende Qualität hinsichtlich Dynamik, Ortsauflösung usw. geachtet wird. Nach der Farbbildgewinnung 16 jedoch sind aus den Farbbildern gewonnene Ergebnisse jederzeit aus den hierzu zwischenzuspeichernden Farbbildern reproduzierbar bzw. überprüfbar.

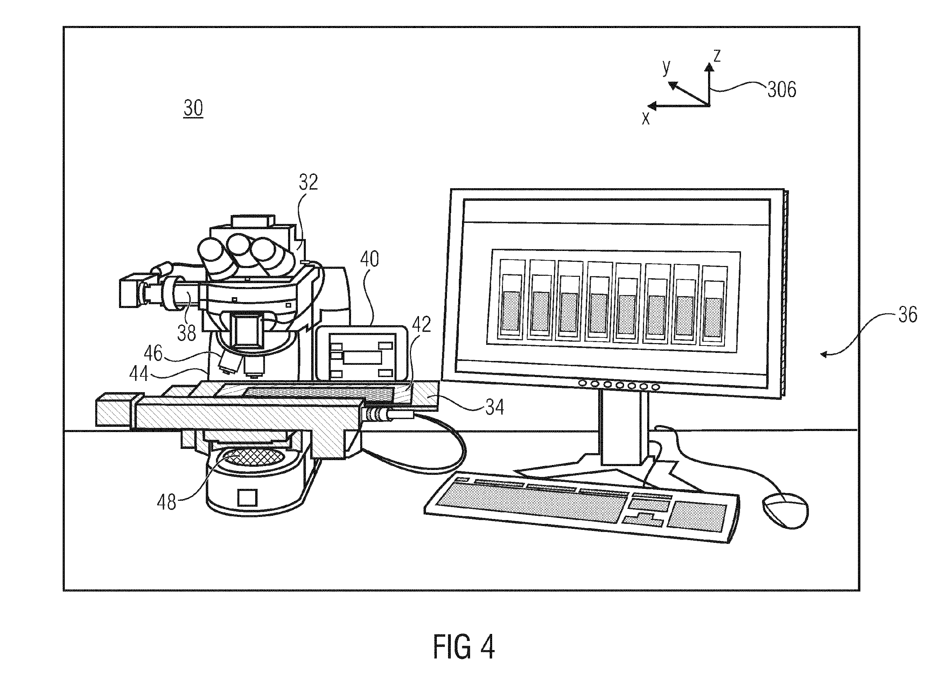

Fig. 3 zeigt ein Verfahren zur Quantifizierung einer Schädigung eines Hautgewebeschnittes gemäß einem Ausführungsbeispiel, das dem Konzept von Fig. 2 folgt. Wie es zu sehen ist, umfasst bei dem Verfahren von Fig. 3 die erste Phase 14 einen Einwirkschritt 20, bei dem der Hautgewebeschnitt oder das Hautgewebe, aus der der Hautgewebeschnitt per histologischem Schnitt erhalten wird, einer einen zerstörenden Einfluss aufweisenden Substanz ausgesetzt wird, sowie einen Schritt 22 des histologischen Schneidens des Hautgewebes, um den Hautgewebeschnitt zu erhalten. Vorzugsweise wird, wie es in Fig. 3 gezeigt ist, der Schritt 22 des histologischen Schneidens nach dem Einwirkschnitt 20 ausgeführt. Das Ergebnis der Phase 14 bzw. des Schrittes 22 ist dann ein oder ein Satz von Hautgewebeschnitten, welcher bzw. welche dann in einem Schritt 24 mittels eines Mikroskops aufgenommen wird/werden, um ein jeweiliges Farbbild zu erhalten. Vorzugsweise liegt bereits die Farbbildaufnahme nach dem Schritt 24 in digitaler Form vor. Eine mögliche Vorrichtung inklusive Mikroskop zur Verwendung in der Durchführung des Schrittes 24 ist in Fig. 4 gezeigt und wird im Folgenden beschrieben.

Dem die Phase 16 repräsentierenden Schritt 24 schließt sich dann ein Schritt 26 der Schädigungsquantifizierung an, welcher Schritt im Folgenden noch detailliert beschrieben wird und der, wenn das bzw. die Farbbilder aus Schritt 24 bereits digital vorliegen, beispielsweise in Form einer Software realisiert sein kann, wobei allerdings natürlich auch eine Hardware-basierte bzw. Programmierbare-Hardware-Lösung denkbar wäre.

Bei dem Verfahren von Fig. 3 können beispielsweise künstliche Hautmodelle für das Hautgewebe verwendet werden. Diese können in dem Schritt 20 partiell mit irritativen Substanzen behandelt werden. Das heißt, einige können unbehandelt bleiben, andere werden behandelt, wobei die Einwirkzeit unter den Proben variieren kann. Beispielsweise erfolgt das Aufbringen von Substanzen auf das Hautgeweben *in vitro,* wobei dann die Substanz für eine definierte Zeit einwirkt. Optional ist es möglich, dass eine bekannte Testsubstanz auf einen Teil des Testgewebes aufgebracht wird, um Chargen-Variationen ausgleichen bzw. erkennen zu können. Die Hautgewebeproben werden beispielsweise aus Human-Zellen gezüchtet und können daher biologische Varianz aufweisen.

In dem Schritt 22 werden die so in Schritt 20 vorbereiteten mehreren Proben geeignet präpariert und histologische Schnitte derselben angefertigt. Sie liegen dann auf einem Objektträger für die Mikroskopie vor. Geeignete Standardverfahren der Pathologie sind in Schritt 22 anwendbar, wie z.B. solche, die in dem Wikipedia-Artikel "Histologie" erwähnt sind.

Zusammenfassend werden also in den Schritten 20 und 22 ein oder mehrere Hautgewebeschnitte erzeugt. Für jeden Hautgewebeschnitt wird in dem Schritt 24 ein Farbbild erzeugt. Dazu kann beispielsweise die Vorrichtung von Fig. 4 verwendet werden. Fig. 4 stellt somit eine Vorrichtung 30 zum Aufnehmen eines Farbbildes des bzw. der Hautgewebeschnitte dar. Die Vorrichtung 30 von Fig. 4 umfasst ein Mikroskop 32 und ein Objektträgerverfahrgerät 34, die ausgebildet sind, um so zusammenzuwirken, dass sich überlappende Einzelbilder des Hautgewebeschnittes erzeugt werden. Die Vorrichtung 30 umfasst ferner eine Einrichtung 36 zum Aneinanderfügen der Einzelbilder, um das jeweilige Farbbild des jeweiligen Hautgewebeschnittes zu erhalten, die in Fig. 4 exemplarisch als ein Arbeitsplatzcomputer dargestellt ist. Die Einrichtung 36 kann allerdings nicht nur in Software implementiert sein, sondern könnte alternativ auch in Hardware oder als programmierbare Hardware implementiert sein.

Insbesondere kann es sich bei dem Mikroskop 32 beispielsweise um ein solches mit einer größeren und einer feineren Vergrößerungseinstellung handeln, zwischen denen ein Objektivwechsler 38 das Mikroskop 32 manuell oder automatisch zu wechseln vermag. Eine Steuereinrichtung 40 der Vorrichtung 30, die in dem Mikroskop 32 untergebracht sein könnte oder zusammen mit der Einrichtung 36 auf einem gemeinsamen Prozessor laufen könnte, kann vorgesehen sein, um das Mikroskop 32 und das Objektträgerverfahrgerät 34 so anzusteuern, dass zunächst eine Übersichtsaufnahme mit der groben Vergrößerung von dem oder den Hautgewebeschnitt(en) auf dem Objektträger 42 erzeugt wird, in welcher Übersichtsaufnahme die Steuereinrichtung 40 dann eine Position des Hautgewebeschnitts auf dem Objektträger 42 unter Auswertung einer beispielsweise bimodalen Verteilung von Grauwerten in dem Übersichtsbild bestimmt, woraufhin die Steuereinrichtung 40 Mikroskop 32 und Objektträgerverfahrgerät 34 so ansteuert, dass ein interessierender Hautgewebeschnitt mit der feineren Vergrößerung an der so bestimmten Position aufgenommen wird, um das Farbbild dieses Hautgewebeschnitts für die darauffolgende Schädigungsquantifizierung 26 (siehe Fig. 3) zu erhalten. Beide Aufnahmen, d.h. Übersichtsaufnahme und Vergrößerungsaufnahme, können durch Aufnahme mehrerer überlappender Einzelbilder mit nachträglicher Aneinanderfügung, wie es im Vorhergehenden beschrieben worden ist, erzeugt werden.

In einer konkreten Implementierung ist es beispielsweise möglich, das Mikroskop 32 so aufzubauen, dass es neben dem Mikroskopstativ 44 einen 6x Objektivrevolver 46 mit codierten Objektivslots sowie eine Halogenlichtquelle 48 aufweist.

Der vorhergehend erwähnte zweistufige Ansatz zur digitalen Abtastung des jeweiligen Hautgewebeschnittes kann folgendermaßen umgesetzt werden. Insbesondere kann der Objektträger 42 zunächst in niedrigerer Auflösung aber dafür mit höherer Geschwindigkeit eingescannt werden, um die Lage und Ausdehnung der Hautschnitte auf dem Objektträger 42 lokalisieren bzw. segmentieren zu können. Details hierzu werden im Folgenden noch näher erläutert. Nach diesem Schritt können dann die entsprechenden, automatisch segmentierten Bereiche des Objektträgers 42 in höherer Auflösung für die anschließende Analyse bzw. Schädigungsquantifizierung eingescannt werden. Hierzu werden durch den Objektivwechsler 38 entsprechende Objektive des Objektrevolvers 46 automatisch in den Strahlengang des Mikroskops 32 eingeschwenkt.

Die optische Auslegung des Mikroskops 32 kann sich an den typischen Größen von Zellkernen orientieren, die mit 5-16 Mikrometer angegeben wird. Beispielsweise kann das Mikroskop 32 eine optische Auflösung von kleiner 20 µm im Feinvergrößerungsmodus aufweisen und eine optische Auflösung von mehr als 20 µm und weniger als 70 µm im Grobvergrößerungsmodus zur Erzeugung des Übersichtsbilds.

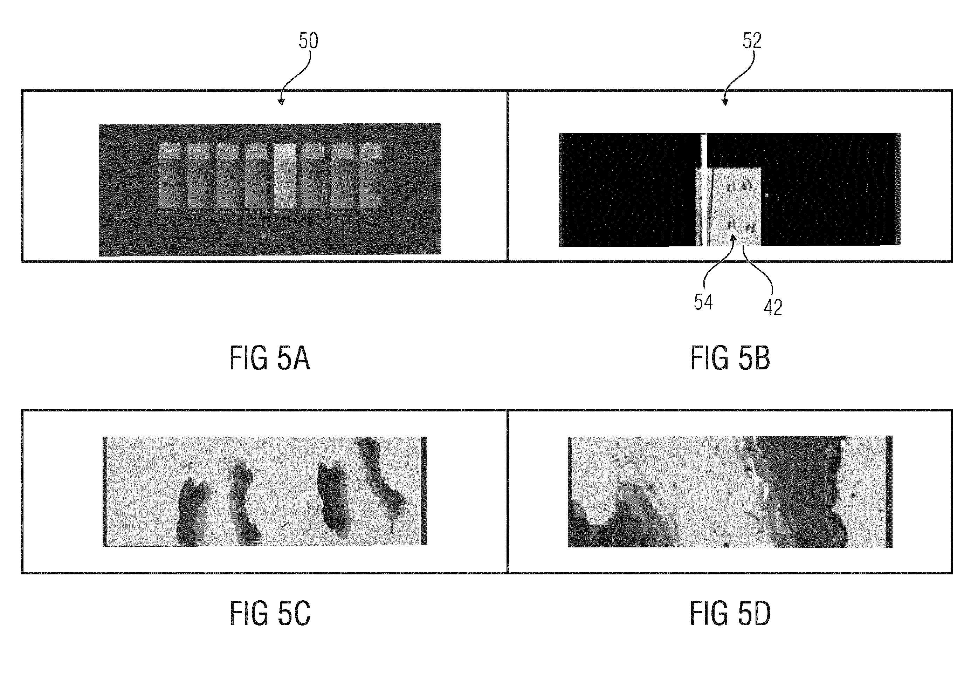

Es ist möglich, die Vorrichtung 30 von Fig. 4 so zu konfigurieren, dass sie in der Lage ist, eine entsprechende Identifikationsinformation auf dem Objektträger 42 automatisch zu erkennen, wie zum Beispiel einen Barcode oder einen anderen maschinenlesbaren Code, so dass eine anschließende Archivierung des Objektträgers 42 und Bewahrung der Zuordnung zu den erzeugten Farbbildern erleichtert wird. Ein Beispiel für eine maschinenlesbare Identifikationsinformation auf einem Objektträger 42 ist exemplarisch in Fig. 5a bei 50 gezeigt. Nach Erkennung der Identifikationsinformation 50 steuert die Steuereinrichtung 40 des Mikroskops 32 beispielsweise so an, dass danach zunächst ein Grobübersichtsbild des Objektträgers 42 wie in Fig. 5b erzeugt wird. In diesem Übersichtsbild 52 sind die Hautgewebeschnitte - in dem Fall von Fig. 5b exemplarisch acht, und exemplarisch mit dem Bezugszeichen 54 angezeigt - leicht detektier- und lokalisierbar. Im Anschluss daran können die lokalisierten bzw. segmentierten Hautgewebeschnitte in einer höheren Auflösung, wie zum Beispiel mit einem 40x-Objekt, eingescannt werden. Fig. 5c zeigt vier nebeneinander liegende Hautgewebeschnitte 54 auf dem Übersichtsbild 52 von Fig. 5b und im Vergleich dazu zeigt Fig. 5d ein Einzelbild aus der Mehrzahl von Feinvergrößerungseinzelbildern, die dann zu dem Farbbild eines jeweiligen einzelnen Hautgewebeschnittes zusammengefügt werden.

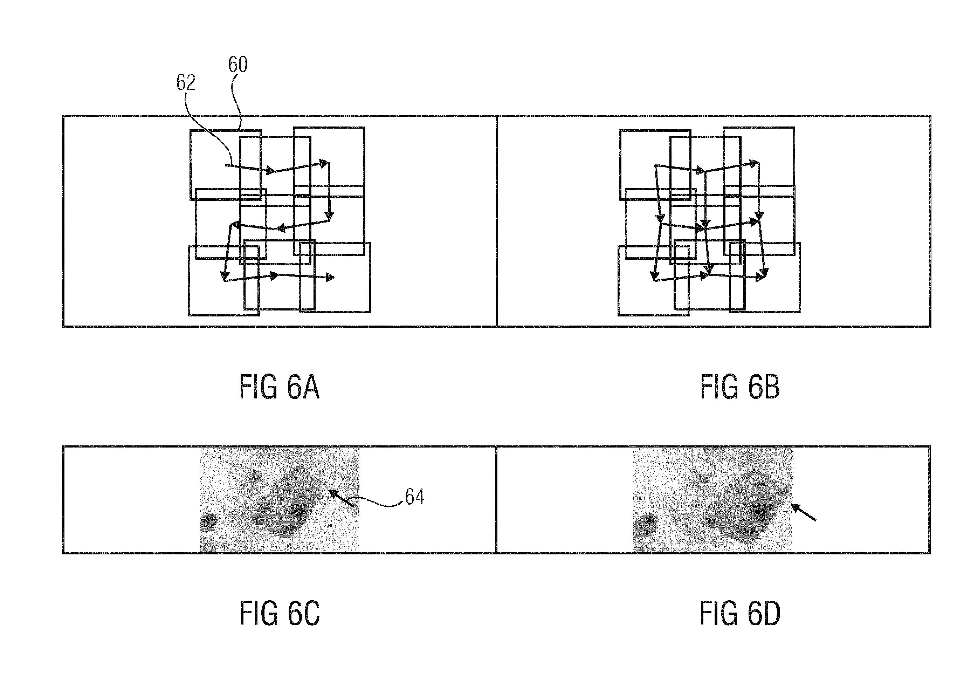

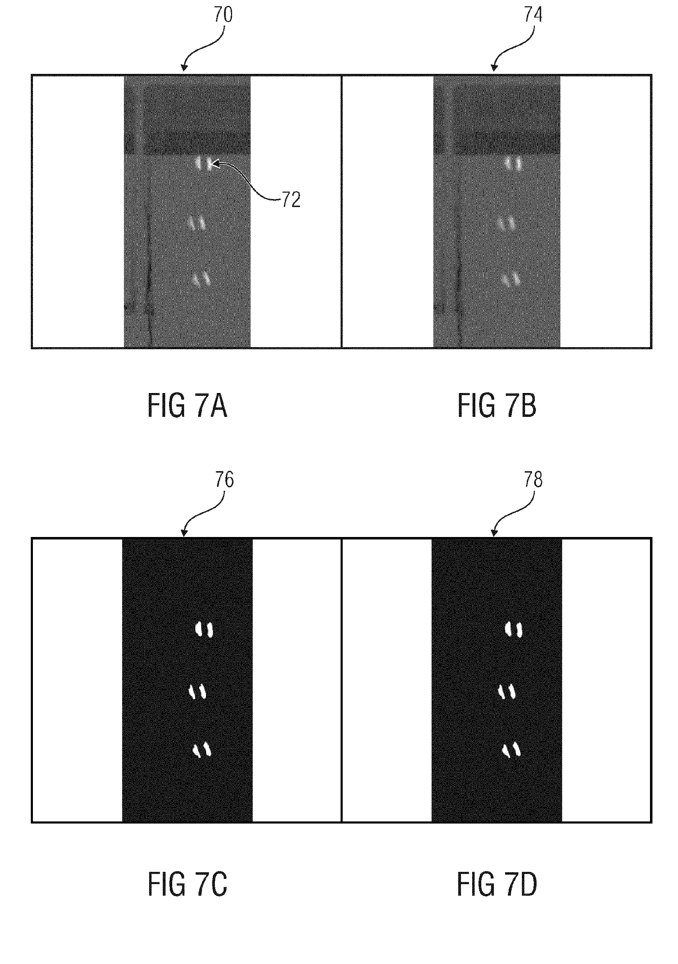

Zur Erzeugung der hochauflösenden Farbbildaufnahme eines interessierenden Hautgewebeschnittes wird beispielsweise ein mäanderförmiges Ablauf des Scans des Gesichtsfeldes des Mikroskops 32 über den interessierenden Hautgewebeschnitt auf dem Objektträger 42 verwendet, wie er exemplarisch in Fig. 6a dargestellt ist. Der Reihe nach werden Einzelbilder 50 mit der feinen Vergrößerung erzeugt, die sich gegenseitig überlappen, wobei die Versatzvektoren 62 zwischen aufeinanderfolgenden Einzelaufnahmen 60 zusammengefügt die Mäanderform ergeben. Obwohl natürlich für jede Einzelnahme 60 ein Aufnahmeort bekannt ist, den das Objektträgerverfahrgerät 34 mit dem Objektträger 42 anfuhr, als die jeweilige Einzelaufnahme 60 erzeugt wurde, sind diese Aufnahmeorte mechanischen Toleranzen unterworfen, so dass auch die Aneinanderfügung der Einzelbilder alleine auf Basis dieser Aufnahmeorte dieser Toleranz unterläge. Vorzugsweise führt deshalb die Einrichtung 36 ein bildbasiertes Zusammensetzen der Einzelbilder 60 aufgrund einer Ähnlichkeitsuntersuchung benachbarter Einzelbilder 60 innerhalb ihrer gegenseitigen Überlappung durch, was auch als Stitching bezeichnet wird. Besonders bevorzugt wird, wenn dabei eine globale Optimierung der Bild-zu-Bild-Registrierung durchgeführt wird, wie es durch Pfeile in Fig. 6b angedeutet ist. Die Figuren 6c und 6d zeigen eine zusammengefügte Gesamtaufnahme eines Objekts auf einem Objektträger, und zwar in dem Fall von Fig. 6c lediglich auf Basis der mechanisch ermittelten Aufnahmeorte, und im Fall von Fig. 6d unter Verwendung der Auswertung der Ähnlichkeitsanalyse benachbarter Einzelbilder durch globale Optimierung. Wie es zu sehen ist, können auftretende Artefakte 64 durch die Ähnlichkeitsanalyse im Überlappungsbereich benachbarter Einzelbilder 60 vermieden werden. Unabhängig davon, ob nun das Übersichtsbild durch Zusammenfügung von Einzelbilder oder durch eine einzige Gesamtaufnahme erhalten worden ist, wird nun beschrieben, wie anhand eines Übersichtsbildes, für welches ein Beispiel in Fig. 7a gezeigt ist, eine Position eines interessierenden Hautgewebeschnittes auf dem Objektträger 42 bestimmt werden kann. Diese Funktionalität ist der Steuereinrichtung 40 inne und kann beispielsweise eine Software-Routine sein, die auf dem gleichen Prozessor abläuft wie die Funktionalität der Aneinanderfügungseinrichtung 36.

In dem Fall einer üblichen Färbung des Hautgewebes im Rahmen der Anfertigung des Hautgewebeschnittes in Schritt 22 (Fig. 3) weisen diejenigen Regionen in dem Übersichtsbild 70, die den Hautgewebeschnitten entsprechen, eine bläuliche Färbung im Vergleich zum Rest des Objektträgers, d.h. zum Hintergrund, auf. Eine einfache Grauwertkonvertierung würde dieser Information nicht gerecht werden. Um nun jedoch bei dem Übergang von dem dreidimensionalen Farbraum in eine eindimensionale Grauwertskalierung in der Grauwertinformation der einzelnen Pixelwerte ein möglichst gutes Maß für die Wahrscheinlichkeit zu haben, ob das Pixel entweder zum Hintergrund oder zu dem Hautgewebeschnitt gehört, wird bei der Grauwertkonvertierung des Farbübersichtsbildes 70 eine Subtraktion des Blaukanals vom Grünkanal durchgeführt, so dass die den Hautgewebeschnitten 76 entsprechenden Bereiche verstärkt werden. Sie erscheinen dann bei der gewählten Grauwertkonvertierung besonders hell, wohingegen der Hintergrund dunkel erscheint. Die gewählte Grauwertkonvertierung erhöht also den Kontrast zwischen Hautgewebeschnitt-Pixeln einerseits und Hintergrund-Pixeln andererseits. Da ein Übersichtsbild 70 üblicherweise recht rauschbehaftet ist, ist es möglich, dass in einem vorhergehenden Schritt die einzelnen Farbkanäle mittels eines Gauß-Filters oder eines ähnlichen Tiefpassfilters geglättet werden, so dass beispielsweise der in der Mitte des entsprechenden Filterkernels befindliche Bildpunkt einen Mittelwert oder ein anderes Maß für die zentrale Tendenz der mit der Kernelfilterfunktion gewichteten Farbwerte innerhalb des Kernels des jeweiligen Farbkanals erhält. Wie bereits erwähnt, zeigt Fig. 7a ein dementsprechendes Übersichtsbild 70 in der entstehenden Grauskalierung, in welchem Bild dermale Bereiche 72 einen hohen Grauwert aufweisen, während der Rest des Objektträgers einen geringen Grauwert aufweist.

Um Unebenheiten zu glätten, kann es sein, dass das Übersichtsbild 70 anschließend noch ein weiteres Mal gefiltert wird, wie zum Beispiel abermals tiefpassgefiltert, wie zum Beispiel mittels eines Gauß-Filters. Die Grenzfrequenz dieses Tiefpassfilters liegt tiefer als bei dem optionalen, auf die einzelnen Farbkanäle angewendeten Tiefpassfilter. Das Ergebnis ist in Fig. 7b gezeigt.

Da Vorder- und Hintergrund des gefilterten Bildes 74 stark unterschiedliche Grauwerte aufweisen, ist das Histogramm des Bildes 74 bimodal. Das erste Maximum und die dazugehörende Häufigkeitsverteilung im unteren Grauskalabereich beinhaltet hauptsächlich den Hintergrund, während der zweite Modus des Grauwerthistogramms den Vordergrund in den helleren Hautgewebeschnitten beinhaltet. Unter Auswertung dieser bimodalen Verteilung von Grauwerten in dem Übersichtsbild 74 - und, falls die Tiefpassfilterung weggelassen wird, dem Übersichtsbild 70 - wird ein Binärbild erhalten, in welchem Bereiche des ersten binären Werts Hautgewebeschnittstellen entsprechen, wohingegen Bereiche des anderen binären Werts dem Hintergrund entsprechen. Die Hautgewebeschnitte aus dem Objektträger sind dann also segmentiert.

Bimodale Histogramme lassen sich beispielsweise automatisiert mit dem Verfahren nach [3] in Vorder- und Hintergrund trennen. Dieses Verfahren approximiert zwei Gauß-Funktionen in das Histogramm. Somit lassen sich die beiden Modi mathematisch beschreiben. In dem zweiten Schritt wird dann der Schnittpunkt zwischen den berechneten Funktionen ermittelt. Dieser Schnittpunkt dient dann als Schwellwert für die sich anschließende Binarisierung des Bildes. Das Ergebnis einer solchen Binarisierung ist in Fig. 7c gezeigt.

Um die Kontur der segmentierten Bereiche der Hautgewebeschnitte in dem Binärbild 76 zu glätten und ggf. vorhandene "Löcher" in dem Segmentierungsergebnis zu füllen, kann es sein, dass das Binärbild 76 abschließend binärmorphologisch geschlossen wird, wobei das Ergebnis eines solchen zusätzlichen optionalen Schrittes in Fig. 7d in Form des Binärbildes 78 gezeigt ist. Die Konturen der einzelnen Dermaschnitte - in dem Fall von Fig. 7a-7d exemplarisch sechs Stück - werden beispielsweise als Punktlisten für die weitere nachfolgende Verarbeitung abgespeichert.

Es sei darauf hingewiesen, dass die soeben beschriebenen Schritte in dem Zusammenhang mit der Lokalisierung bzw. Segmentierung der Hauptgewebeschnitte sowohl in der Auflösung des Übersichtsbilds als auch in der feinen Auflösung stattfinden kann. Das bedeutet also insbesondere, dass die vorerwähnten hochauflösenden Farbbilder der Hautgewebeschnitte letztere in Gruppen gemeinsam in einem Farbbild anzeigen können, so dass die Verarbeitungsschritte gleichzeitig zur Segmentierung und Lokalisierung von mehreren Hautgewebeschnitten führen.

Insofern stellen die soeben beschriebenen Schritte aus Fig. 7 auch einen optionalen Vorbereitungsschritt des nachfolgend beschriebenen Ausführungsbeispiels für eine Umsetzung des Schädigungsquantifizierungsschrittes 26 dar.

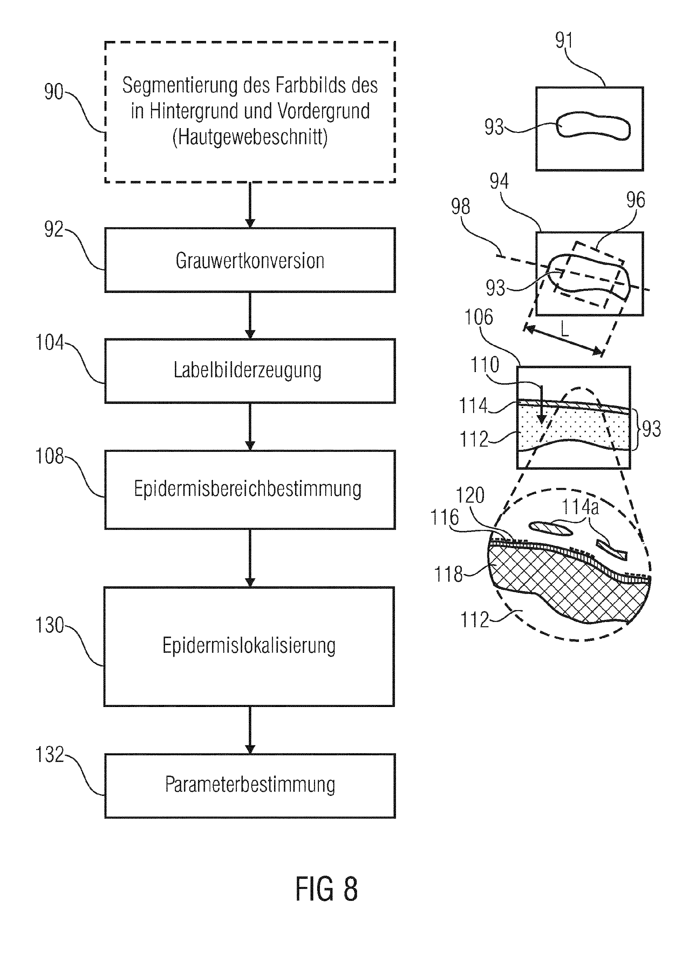

Fig. 8 zeigt ein Verfahren zur Schädigungsquantifizierung gemäß einem Ausführungsbeispiel. Die Schritte in Fig. 8 können einzelnen Einrichtungen einer entsprechenden Vorrichtung entsprechen. Sie können insbesondere einzelnen Routinen in einer Softwareumsetzung entsprechen, welche Software beispielsweise auf dem gleichen Prozessor abläuft wie die Steuereinrichtung 40 und/oder die Aneinanderfügungseinrichtung 36 aus Fig. 4. Wie es also in Fig. 8 zu sehen ist, besteht ein optionaler erster Schritt 90 in einer Segmentierung des Farbbilds des Hautgewebeschnittes in Hintergrund und Vordergrund, d.h. Pixel, die nicht den interessierenden Hautgewebeschnitt zeigen und Pixel, die selbigen darstellen. Die Verarbeitung kann wie im Vorhergehenden erwähnt erfolgen, d.h. mittels Grauwertkonvertierung und anschließender Auswertung der bimodalen Verteilung zum Erhalt des Binärbildes.

Ziel der nachfolgenden Verarbeitung ist nun die Schädigungsquantifizierung wozu, wie es bereits in der Beschreibungseinleitung der vorliegenden Anmeldung erwähnt worden ist, erforderlich ist, aus dem Farbbild die Epidermis zu segmentieren, da diese Aufschluss über das Ausmaß der Schädigung liefert.

Wenn also der nächste Schritt 92 des Verfahrens von Fig. 8 beginnt, ist zumindest schon eine gewisse Information vorhanden, nämlich dass sich in dem Farbbild 91, das der weiteren, mit dem Schritt 92 beginnenden Verarbeitung unterzogen wird, der Bereich mit dem Hautgewebeschnitt, d.h. der Bereich 93, an einer ungefähren Stelle befindet oder relativ formatfüllend befindet. In dem Schritt 92 wird nun zunächst eine Konvertierung des Farbbilds 91 in ein Grauwertbild 94 durchgeführt, wobei die Grauwertkonversion aus Schritt 92 anders sein kann als die im Vorhergehenden beschriebene optionale stattfindende Grauwertkonversion zur Lokalisierung. Insbesondere versucht die Grauwertkonversion aus Schritt 92 als Grundlage für den nächsten Schritt dienen zu können, bei welchem nicht nur zwischen Hintergrund und Hautgewebeschnitt unterschieden werden soll, sondern eine Unterscheidung in drei Gebiete bzw. Labels, nämlich Hintergrund, Dermis und Epidermis. Würde beispielsweise für die Grauwertkonversion aus Schritt 93 eine übliche Grauwertkonversion verwendet werden, würden Aufnahmeartefakte, Fingerabdrücke oder Substanzen das spätere Schädigungsquantifizierungsergebnis stören können. Für die Farbwertkonversion aus Schritt 92 kann daher beispielsweise vorteilhafterweise eine gewichtete Mittelung der Farbkanäle in das Grauwertbild 94 verwendet werden, bei welcher Transformation der Blau-Kanal mit a, und die Rot- und Grün-Kanäle mit jeweils bᵣ und bg gewichtet werden. Beispielsweise beträgt a = 0,75 und b_{r,g} = 0,15. Andere Transformations- bzw. Gewichtungsverhältnisse wären allerdings ebenfalls denkbar, wobei a beispielsweise zwischen 0,6 und 0,85, jeweils einschließlich, und b_{r,g} zwischen 0,7 und 0,27, jeweils einschließlich, liegt. Der Grauwert GRAU ergäbe sich durch GRAU = a·BLAU + bᵣ·ROT + b_{g}·GRÜN. Durch eine solche Skalierung bzw. Abbildung von dem dreidimensionalen Farbraum des Farbbildes 91 in die Grauwertskala des Grauwertbildes 94, werden Violett-Töne der optional verwendeten HE-Färbung bei der Vorbereitung der Hautgewebeschnitte in Schritt 22 besonders betont.

Als ein vorbereitender Schritt zu Schritt 92 und den nachfolgenden Schritten, die noch beschrieben werden, kann es sein, dass entweder automatisch oder manuell durch den Benutzer ein der nachfolgenden Verarbeitung in den Schritten 92 und den nachfolgenden Schritten zugrunde zu legender Ausschnitt 96 aus dem Farbbild 91 bzw. dem Grauwertbild 94 aussondiert wird, um Bereiche am Rand des Hautgewebeschnittgebietes 93 von der Verarbeitung auszuschließen, die zu Verfälschungen des Schädigungsquantifizierungsergebnisses führen könnten. Ein Hautgewebeschnitt besitzt beispielsweise üblicherweise eine Längserstreckungsrichtung 98, entlang der sich die Epidermis erstreckt, und zwar insbesondere vornehmlich auf einer Seite der Längserstreckungsrichtung 98, da sie ja den Hautgewebeschnitt 93 an dieser Seite des Hautgewebeschnittes 93 die darunterliegende Dermis abschließt. Der Bereich 96 könnte nun so gewählt werden, dass beispielsweise ausgehend von einer Länge L des Hautgewebeschnittes 93 entlang der Längserstreckungsrichtung 98 lediglich ein innerer Ausschnitt 96 ausgewählt wird, der um 10-20% der Länge L vom jeweiligen Extremalpunkt der Ausdehnung des Hautgewebeschnittes 93 entlang der Längserstreckungsrichtung 98 entfernt ist, so dass der Ausschnitt 96 entlang dieser Richtung nur noch 60 bis 80% der Länge besitzt.

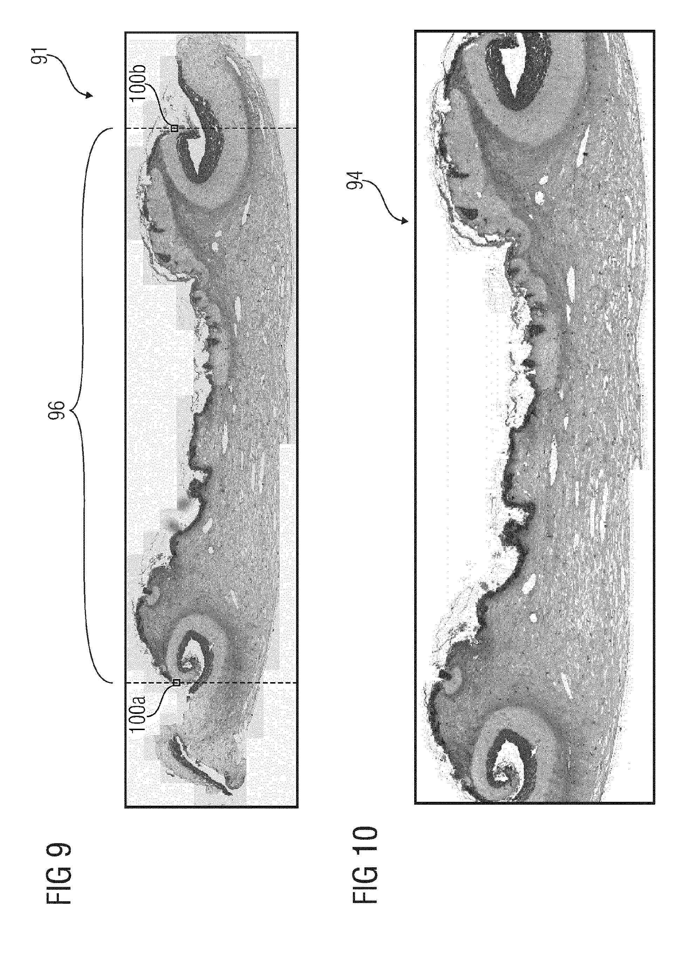

Fig. 9 zeigt die manuelle Definition eines solchen Ausschnitts 96 in dem Farbbild 91 manuell durch den Benutzer durch Vorgabe von sogenannten Saatpunkten 100a bzw. 100b. Durch die Vorgabe dieser Saatpunkte für die nachfolgende Segmentierung und Charakterisierung definiert der Benutzer somit die für die Untersuchung relevante Epidermisregion.

Die Saatpunkte 100a, 100b begrenzen nach dem Ausführungsbeispiel von Fig. 9 den linken und rechten Rand der dazwischen liegenden zu untersuchenden Region 96 entlang der Längserstreckungsrichtung.

Fig. 10 zeigt das Ergebnis der Grauwertkonversion 92 - innerhalb des Abschnitts 96. Als Teil des Schrittes 92 ist das Grauwertbild noch mit einem Glättungsfilter behandelt worden, wozu beispielsweise ein Diffusionsfilter nach Perona-Malik [4] verwendet werden kann. Das Glättungsfilter sollte möglichst kantenerhaltende Eigenschaften aufweisen. Die Haupteigenschaft des Perona-Malik-Filters besteht dementsprechend auch darin, dass die Kanten zwischen Regionen erhalten bleiben und nur innerhalb der Regionen geglättet wird.

In einem nächsten Schritt 104 wird nun Bildpunkten des Grauwertbildes 104 einem jeweiligen Label aus Hintergrund, Epidermis oder Dermis zugewiesen, und zwar unter Verwendung eines Bildverarbeitungsalgorithmus, um ein Labelbild 106 zu erhalten. Am rechten Rand von Fig. 8 ist das Labelbild 106 vereinfacht dargestellt. Hintergrund entspricht den weißen Stellen, die Dermis dem gepunkteten Bereich und die Epidermis dem schraffierten Bereich. Als Bildverarbeitungsalgorithmus kann beispielsweise ein solcher verwendet werden, der u.a. ein Clustering aufweist, d.h. einen iterativen Prozess, der beispielsweise unter Verwendung eines bestimmten Abstandsmaßes unter den Grauwerten der Bildpunkte des Grauwertbildes 94 Bildpunkte so zu drei Clustern bzw. Gruppen zusammenfasst, dass die Grauwerte dieser Bildpunkte zu ihrem jeweiligen Mittelwert gemäß dem gewählten Abstandsmaß einen minimalen Abstand besitzen bzw. z.B. die Summe aller Abstände aller Bildpunkte bezogen auf dieses Abstandsmaß minimal ist.

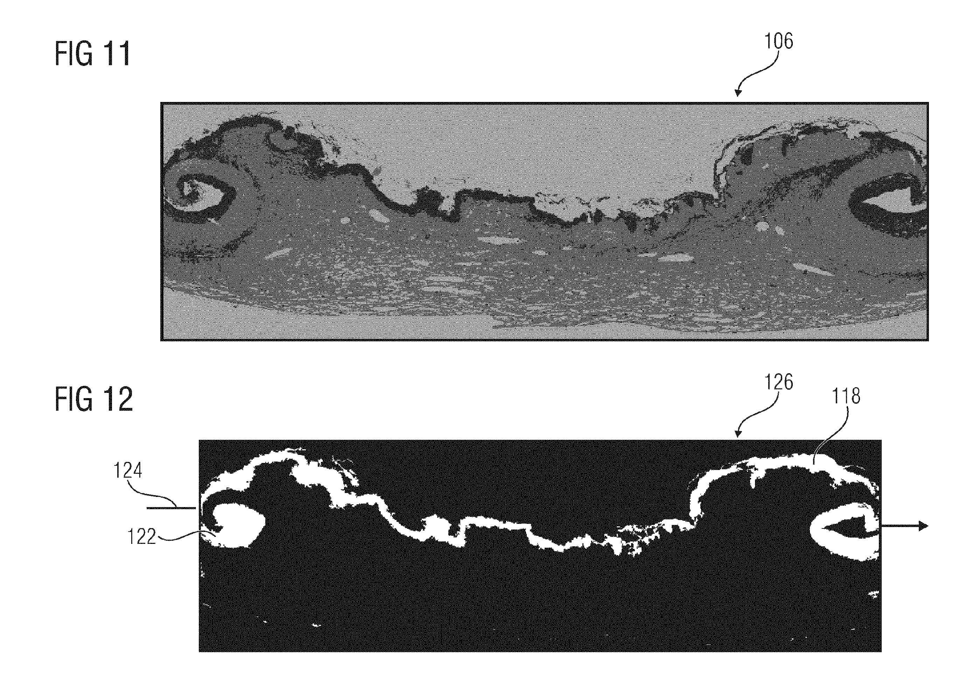

Insbesondere ist es beispielsweise möglich, dass für die Segmentierung ein Schritt 104 ein Fuzzy C-Mean Algorithmus verwendet wird, wie er beispielsweise in [5] beschrieben wird. Der Fuzzy C-Mean Algorithmus ist ein Beispiel für einen Fuzzy-Cluster Algorithmus. Alternativ könnte auch ein K-Means Algorithmus verwendet werden. Der Fuzzy C-Mean Algorithmus liefert reproduzierbare und gute Segmentierungsergebnisse und erfordert keine Interaktion nach einer ersten Einstellung. Die Anwendung des Fuzzy C-Mean Algorithmus auf das Grauwertbild 94 von Fig. 10 ist in Fig. 11 dargestellt. Fig. 11 zeigt also ein tertiäres Labelbild 106. Bildpunkte, die der Epidermis zugeschrieben werden, sind dunkel dargestellt, Bildpunkte, die dem Hintergrund zugeschrieben werden, hell, und Bildpunkte, die der Dermis zugeschrieben werden, in einer mittleren Graustufe.

Das Verfahren von Fig. 8 fährt nun mit einem Schritt 108 fort, bei welchem entlang einer Richtung 110, die von einer der Dermis 112 abgewandten Seite auf die Epidermis 114 des Hautgewebeschnittes 93 weist, ein Außenoberflächenrand 116 der Epidermis 114 bestimmt und ein Füllalgorithmus von dem Außenoberflächenrand 116 aus auf das Labelbild 106 angewendet wird, um einen mit dem Außenoberflächenrand zusammenhängenden und sich von dem Außenoberflächenrand 116 in der Richtung 110 zur Dermis 112 hin erstreckenden Epidermisbereich zu bestimmen, welcher im Unterschied zu dem Epidermisbereich 114 des Labelbildes 106 in Fig. 8 kreuzschraffiert 118 dargestellt ist. Ziel des Schrittes 108 ist es also, aus dem Labelbild 106 den Epidermisbereich 118 so zu bestimmen, dass er zerfranste vorgelagerte Bereiche 114a der Epidermis aussondert, so dass sie bei der nachfolgenden Analyse nicht stören. Der Schritt 108 kann beispielsweise folgendermaßen detailliert ausgeführt werden. Beispielsweise wird zunächst eine äußere Kante 120 des Epidermislabelbereichs 114 aus dem Labelbild 106 bestimmt, d.h. die in Richtung 110 der Dermis 112 abgewandte Kante, die durch die in der Richtung 110 betrachtet zuerst anzutreffenden Pixel gebildet wird. Diese Kante verläuft natürlich unstetig aufgrund der soeben erwähnten Zerfransungen 114a, die beispielsweise auf Artefakte und/oder Epidermis-Schnittreste zurückzuführen sind. Von dieser Kante 120 aus beginnend wird nun ein Füllalgorithmus so angewendet, dass Epidermislabelstellen, die eine gewisse Größe unterschreiten, verschwinden, wie z.B. die Stellen 114a, und kleinere Lücken innerhalb des geschlossenen Bereichs 118 gefüllt werden, wobei wiederum vereinzelte in der Dermis liegende Epidermisinseln nicht mit in den Epidermisbereich anwachsen, da der Füllalgorithmus von der Außenkante aus beginnt.

Dabei kann bei der Bestimmung des Außenoberflächenrands 116 und bei Anwendung des Füllalgorithmus in Schritt 108 beispielsweise ein morphologischer Operator zur Anwendung kommen, um die besagten Artefakte und/oder Epidermis-Schnittreste 114a auf einer der Dermis 112 abgewandten Seite der Epidermis 114 bei der Bestimmung des Außenoberflächenrands 116 auszublenden, so dass der Außenoberflächenrand glatter und näher entlang der Dermis 112 verläuft.

Eine konkrete Ausführung des Schrittes 108 auf das Labelbild von Fig. 11 ist im Ergebnis in Fig. 12 dargestellt. Fig. 12 zeigt also ein Binärbild des Epidermisbereichs 118. Er wurde aus Fig. 11 erhalten, indem die äußere Kante des Hautgewebeschnittes auf der Außenseite der Epidermis berechnet und mit der segmentierten Epidermis verbunden wurde, wobei dabei morphologische Operatoren und ein Füllalgorithmus auf das Bild angewendet wurden.

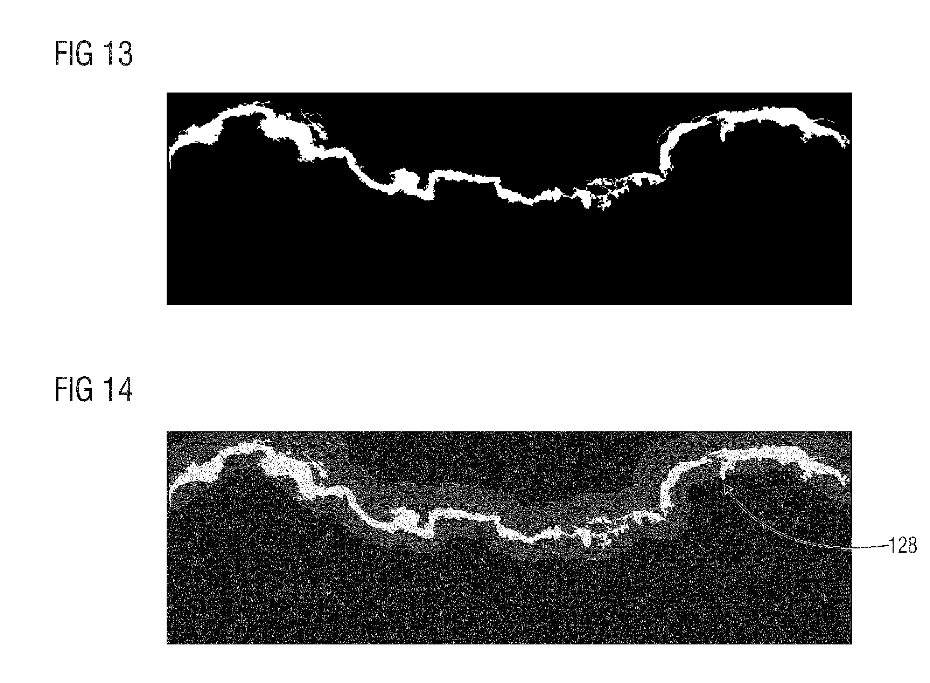

Im Folgenden werden Bezug nehmend auf die Figuren 13 und 14 noch mögliche zusätzliche Schritte beschrieben, die innerhalb der Epidermisbereichsbestimmung 108 durchgeführt werden könnten, aber insgesamt fakultativ sind. Beispielsweise erkennt man in Fig. 12, dass der Epidermisbereich 118 in Wirklichkeit zur Epidermis benachbarte Regionen 122 aufweist, die von der nachfolgenden Verarbeitung ausgeschlossen werden müssen. Sie entstehen seitlich in Längserstreckungsrichtung 124 der Epidermis aufgrund einer seitlichen Einwirkung der Substanz auf das Hautgewebe bzw. aufgrund der Präparation des Hautgewebeschnittes und verfälschen ansonsten die Schädigungsquantifizierung. Die Notwendigkeit der nachfolgend beschriebenen Schritte kann aber ohnehin vermieden werden, indem der Ausschnitt 96 bzw. die Saatpunkte 100 bereits geeignet gewählt werden. Die Abtrennung der Bereiche 122 kann in zwei Stufen erfolgen. Beispielsweise wird es das Binärbild 126 des vorläufigen Epidermisbereiches 118 aus Fig. 12 zunächst analysiert, um zusammengehörige Regionen zu erkennen, zusammenzufassen und zu indizieren. Daraufhin können die Regionen 120 extrahiert werden, nämlich diejenigen Regionen, die mit der Außenkante des Bildes 126 verbunden sind, d.h. den Außenkanten des Bildes, die in Längserstreckungsrichtung 124 einander gegenüberliegen.

Das Ergebnis der Entfernung der entsprechenden Regionen ist in Fig. 13 ersichtlich. Für einen nachfolgenden Schritt können dann Auswüchse 128 mit Hilfe einer Distanztransformation von dem Epidermisbereich 118 abgetrennt werden, d.h. Abschnitte des vorläufigen Epidermisbereiches 118, die von einem sich entlang der Längserstreckungsrichtung 124 des Epidermisbereichs 118 erstreckenden Skelettierungsergebnis des Skelettierungsalgorithmus weiter als ein vorbestimmtes Maß entfernt liegen. Insbesondere wird beispielsweise die äußere Kante, d.h, die der Richtung 110 entgegengewandte Kante, des vorläufigen Epidermisbereichs als Basis für die Berechnung einer Distanztransformation verwendet, während für die Region bzw. den vorläufigen Epidermisbereich 118 selbst durch einen Skelettierungsalgorithmus eine Skelettierung bestimmt wird. Der maximale Skeletonwert (Distanz) kann beispielsweise mit 2,5 multipliziert werden und als Stellwert für die Distanztransformation dienen, wodurch sich in Fig. 14 exemplarisch der "Auswuchs" 128 ergibt, der dann von dem Epidermisbereich 118 abgezogen wird.

In anderen Worten ausgedrückt kann bei der Epidermisbereichsbestimmung ein Skelettierungsalgorithmus auf den Epidermisbereich angewendet werden, um Auswüchse des Epidermisbereichs 118, die von einem sich entlang einer Längserstreckungsrichtung 124 des Epidermisbereichs 118 erstreckenden Skelettierungsergebnis des Skelettierungsalgorithmus weiter als ein vorbestimmtes Maß entfernt liegen.

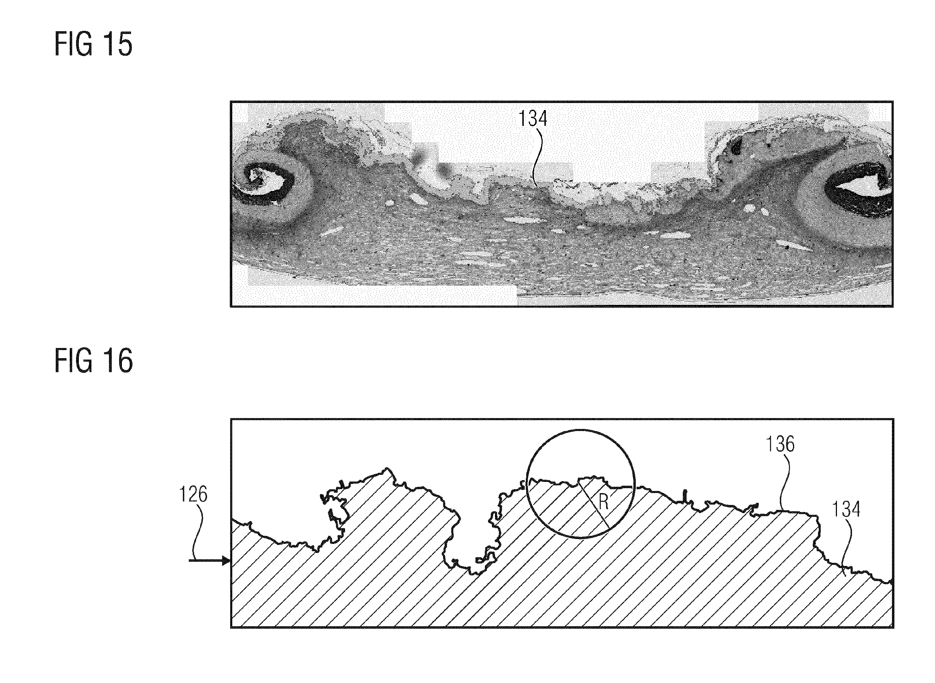

Ein nächster Schritt in dem Verfahren von Fig. 8 ist nun eine Lokalisierung der Epidermis in dem Labelbild 106 unter Verwendung des bestimmten Epidermisbereichs 118. Wie im Vorhergehenden beschrieben sorgt der von der Kante 116 ausgehende Füllalgorithmus dafür, dass sich der Epidermisbereich 118 als relativ geschlossener Bereich von der Kante 116 aus zur Dermis 112 hin erstreckt. Artefakte innerhalb der Dermis 112 gehören somit nicht zum Epidermisbereich, was zu falschen Quantifizierungsergebnissen führen würde. Allerdings soll die Beschaffenheit der Epidermis an sich mit in die Schädigungsquantifizierung einfließen, so dass bei dem Schritt 130 beispielsweise genau diejenigen Bildpunkte des Labelbildes 106 als zur Epidermis gehörig ausgewählt werden, die in dem Epidermisbereich 118 liegen, d.h. Bildpunkte, die in dem Labelbild 106 dem Label der Epidermis zugewiesen sind und gleichzeitig in dem Bereich 118 liegen. Das Ergebnis ist für den sich aus Fig. 13 ergebenden Epidermisbereich und das Labelbild von Fig. 11 in Fig. 15 gezeigt. Der schwarze bzw. grüne Bereich zeigt den Bereich der lokalisierten Epidermis an. Bei diesem Schritt werden also die in den früheren Schritten geschlossenen Löcher der Epidermis wieder aufgemacht.

In einem nachfolgenden Schritt 132 erfolgt eine Parameterbestimmung, in welcher Parameter bestimmt werden, die charakteristische Eigenschaften einer Form der lokalisierten Epidermis beschreiben, d.h. des schwarzen Bereichs 134 in Fig. 15. Wie es im Folgenden noch näher beschrieben wird, können folgende Parameter in Schritt 133 berechnet werden:
a) eine Ausdehnung bzw. eine Anzahl von Bildpunkten der lokalisierten Epidermis in der bzw. entlang der von der der Dermis abgewandten Seite auf die Epidermis der Hautgewebeschnitt weisenden Richtung für unterschiedliche Positionen entlang einer Längserstreckungsrichtung der Epidermis;
b) eine Länge einer der Dermis abgewandten Epidermiskante der lokalisierten Epidermis, soweit die Epidermis innerhalb eines Kreises mit einem vorbestimmten Radius um eine jeweilige Position entlang der Epidermiskante liegt, für unterschiedliche Positionen entlang der Epidermiskante;
c) eine Anzahl von Überschneidungen einer der Dermis abgewandten Epidermiskante der lokalisierten Epidermis mit einem Kreis mit dem vorbestimmten Radius um eine jeweilige Position entlang der Epidermiskante für unterschiedliche Positionen entlang der Epidermiskante;
d) ein Histogramm einer Verteilung einer Länge einer der Dermis abgewandten Epidermiskante der lokalisierten Epidermis, soweit die Epidermis innerhalb eines Kreises mit einem vorbestimmten Radius um eine jeweilige Position entlang der Epidermiskante liegt, für Positionen entlang der Epidermiskante; und

Einzelheiten hierzu, d.h. zu der in Schritt 132 erfolgenden Charakterisierung der Epidermis, gemäß einer konkreten Implementierung, werden nun im Folgenden beschrieben. Diese nachfolgenden Erläuterungen sollen so verstanden werden, dass sie einzeln als Konkretisierung der Bestimmung der Parameter a)-e) der soeben gemachten Auflistung dienen können.

Schädigungen der Epidermis aufgrund der toxischen Einwirkung von Substanzen machen sich durch eine Erosion der epidermalen Schicht bemerkbar. Die geschlossene Kontur wird dabei unterbrochen, die Schichtung dünner. Um diese Auswirkungen zu quantifizieren werden im folgenden verschiedene Parameter beschrieben. Die Charakterisierung erfolgt hierbei anhand der Analyse des Segmentierungsergebnisses, das wie oben beschrieben erstellt wird, und insbesondere der äußeren Epidermiskante (Fig: 16). Da hierbei über die Länge des zu untersuchenden Epidermisabschnitts (eingegrenzt durch beispielsweise die beiden initial gesetzten Saatpunkte 100a,b) eine Vielzahl von Parametern extrahiert wird, werden jeweils die Statistiken Minimum, Maximum, Mittelwert und Standardabweichung zur kompakten Darstellung errechnet.

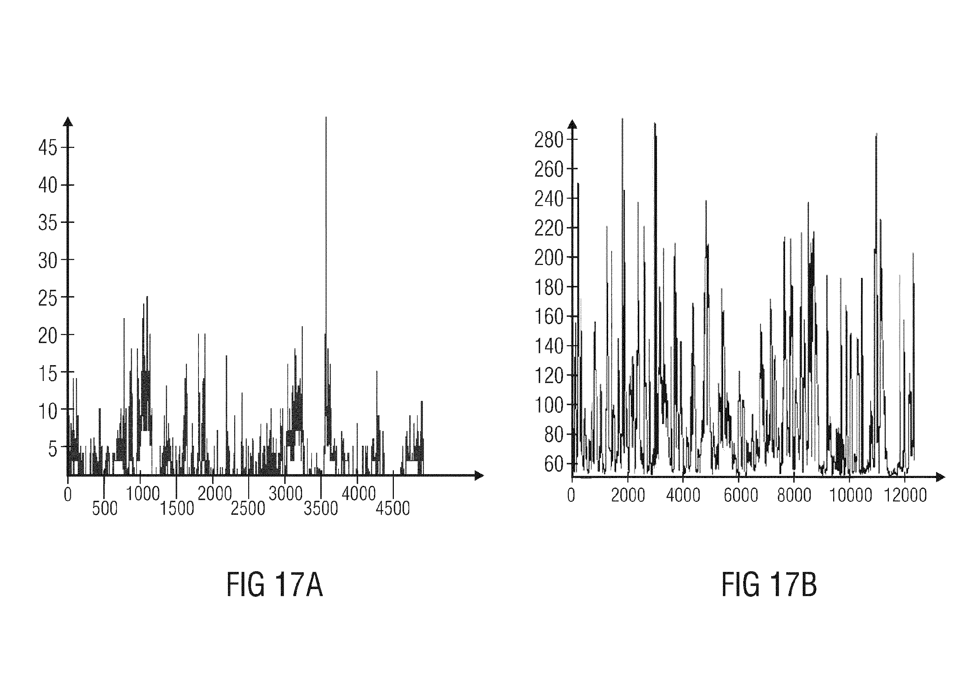

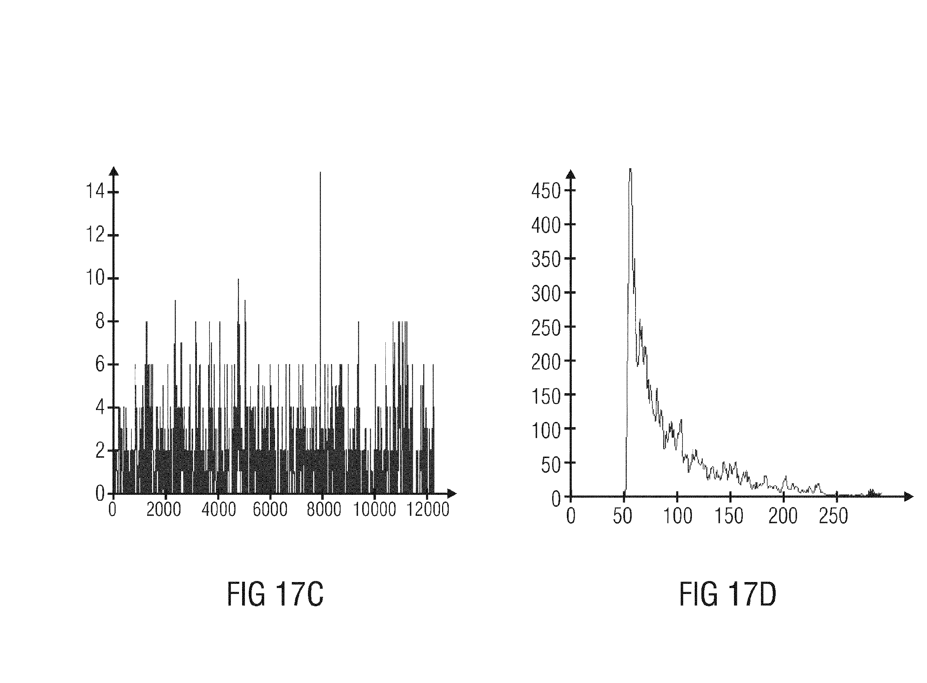

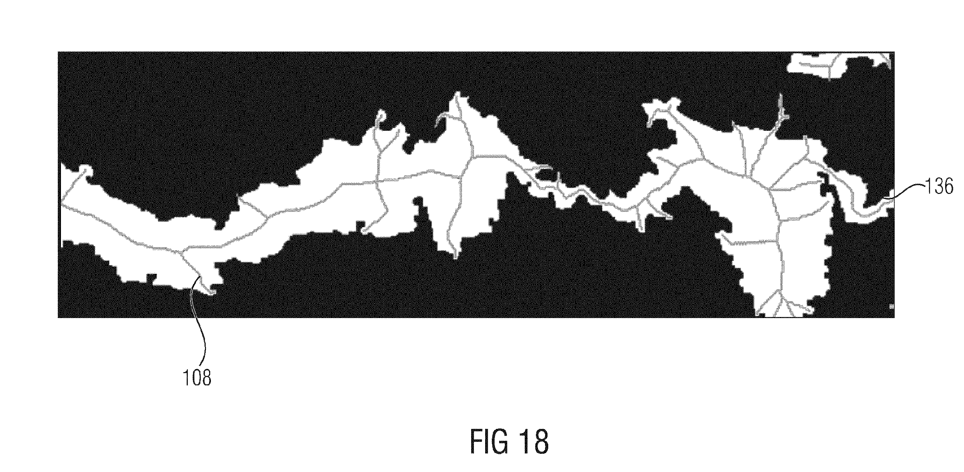

Folgende Parameter können aus dem Segmentierungsergebnis berechnet werden (oder eine Teilmenge daraus):
- Parameter 1: Die Anzahl der übereinanderliegenden Pixel in einer Spalte (Fig. 17a). Bei der Auswertung dieses Parameters wird beispielsweise der gesamte Umfang der Fläche der lokalisierten Epidermis 134 verwendet, d.h. auch die der Dermis zugewandten Umfangskanten, und die Umfangskanten, die Lücken in der lokalisierten Epidermis 134 einschließen. Natürlich wäre es auch ebenfalls möglich, nicht nur die Umfangskanten zu verwenden, sondern alle Pixel in der Epidermis.
- Parameter 2: Für jedes Pixel an der äußeren Kante 136 wird die Länge des Kantensegments innerhalb eines Kreises mit Radius R berechnet Fig. 16 und Fig. 17b). Hier könnte umgekehrt der Parameter 2 auch für jedes Pixel entlang der gesamten Umfangskante der lokalisierten Epidermis 134 durchgeführt werden.
- Parameter 3. Anzahl der Überschneidungen der Kante 136 mit dem Kreis mit Radius R (Fig. 17c). Hier könnte umgekehrt der Parameter 2 auch für jedes Pixel entlang der gesamten Umfangskante der lokalisierten Epidermis 134 durchgeführt werden.
- Parameter 4: Histogramm von Parameter 2 (Fig. 17d).
- Parameter 5: Es wird eine Skelettierung des Segmentierungsergebnisses 134 generiert. Jeder Skelettast entspricht einem Auswuchs (Fig. 18). Es wird die Anzahl der Äste 138 in der Nähe der äußeren Epidermiskante 136 berechnet.

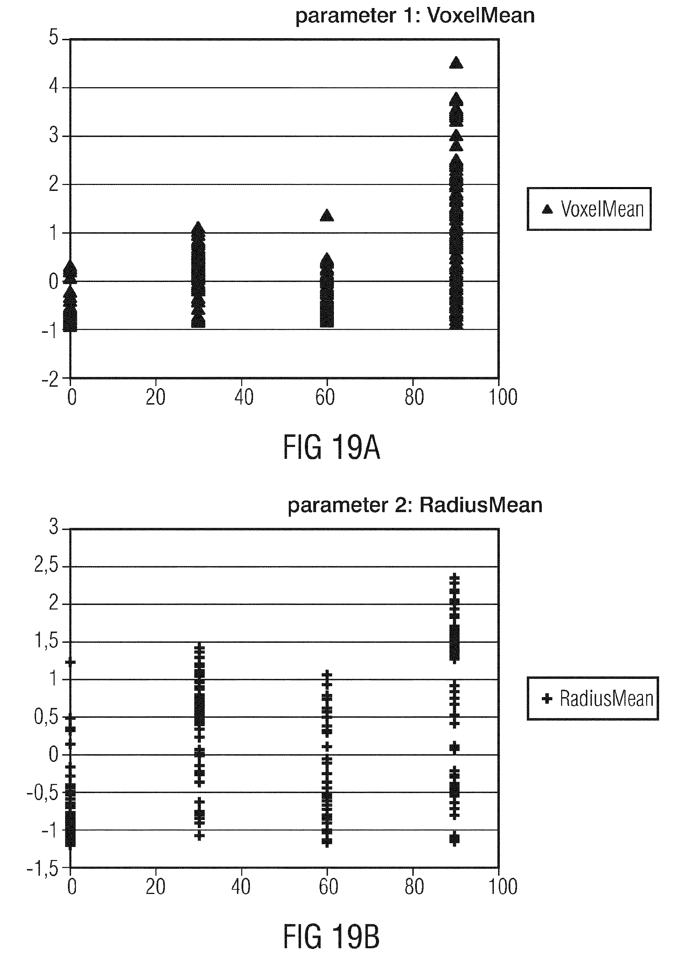

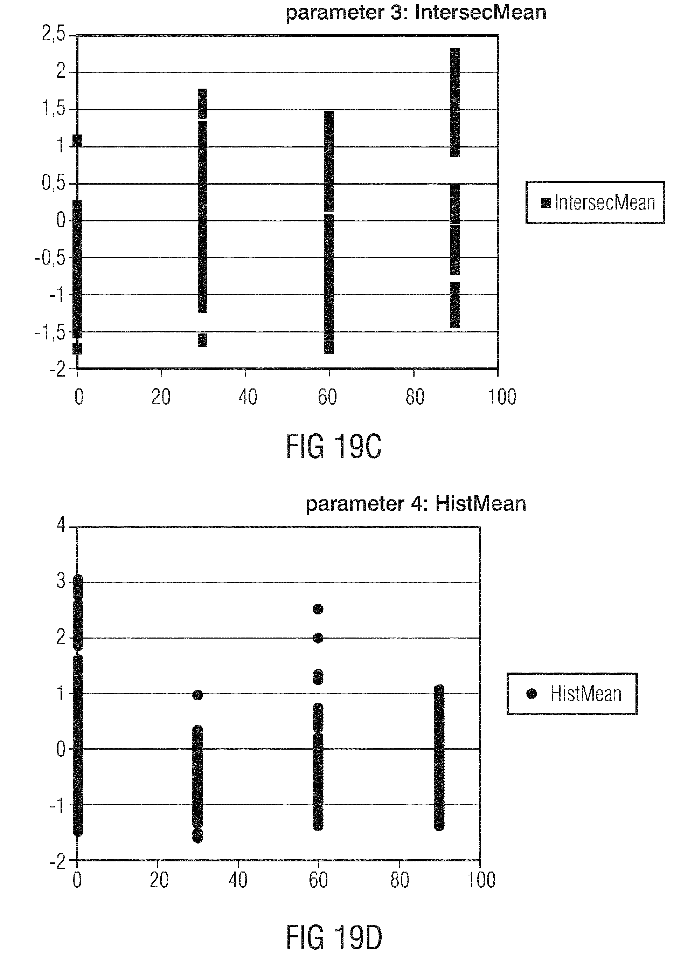

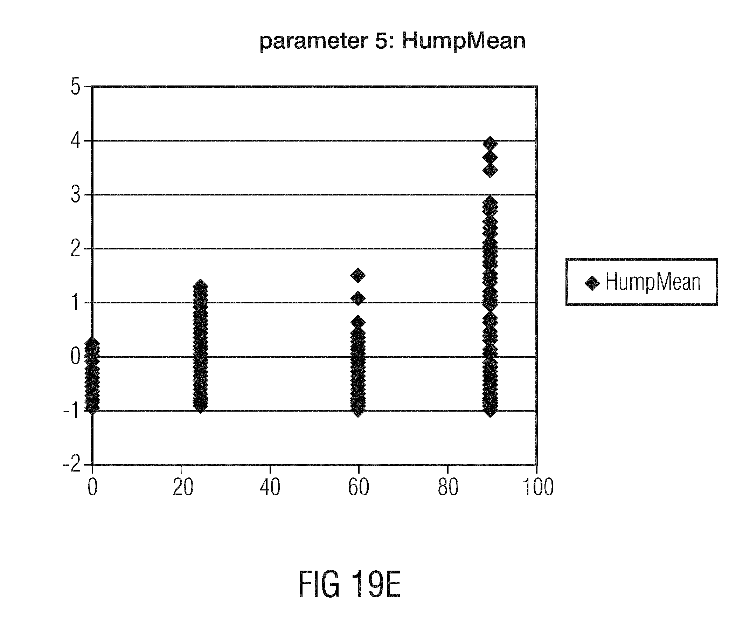

Die oben beschriebenen Parameter 1-5 wurden über die Bilddaten der Referenzstichprobe aus Fig. 1 berechnet und sind im Folgenden dargestellt. Unterschieden werden hierbei unbehandelte Proben, sowie Proben unter Einwirkdauern von 30, 60 bzw. 90 Sekunden. Fig. 19 illustriert die Mittelwerte der jeweiligen Parameter, aufgetragen über die Einwirkdauern. Ganz offensichtlich ist keiner der aufgeführten Parameter für sich alleine trennscharf hinsichtlich der toxischen Schädigung des Gewebes, daher wurden im folgenden Kombinationen aus Parametern evaluiert.

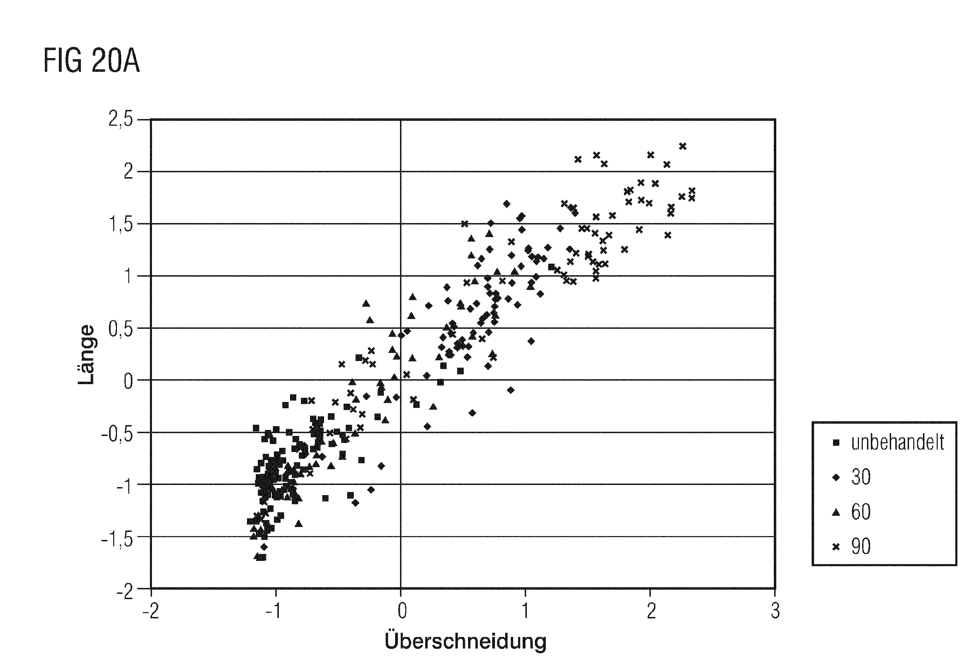

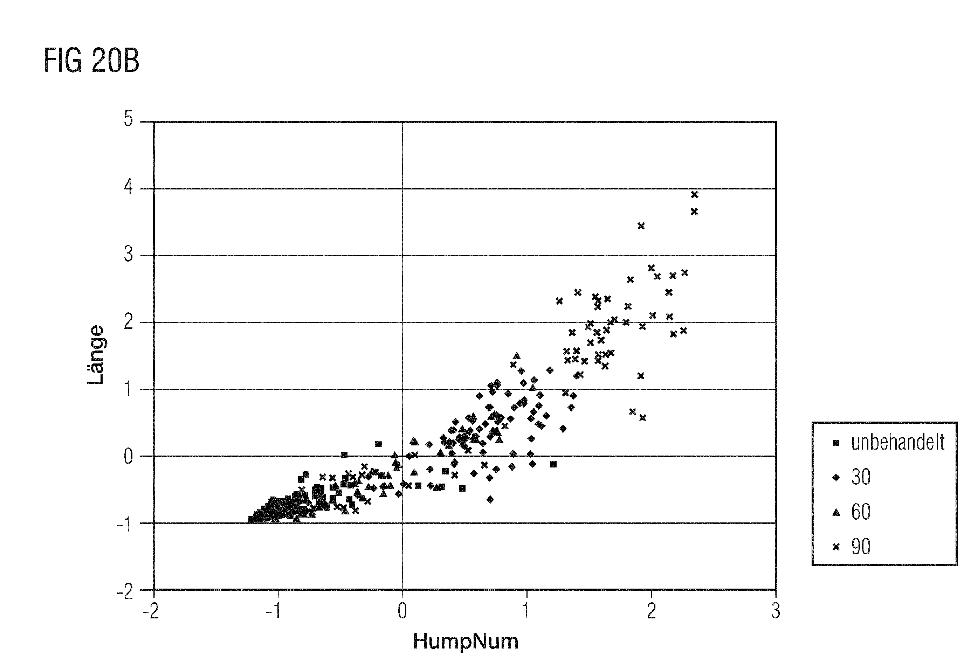

Zur Illustration werden hier nur zwei ausgewählte Parameterkombinationen aufgeführt und in Fig. 20 dargestellt. Deutlich sichtbar zeichnen sich Cluster, also Häufungen, der Datenpunkte der jeweiligen Klassen (unbehandelt, 30, 60, 90) in beiden Kombinationen ab. Ebenso ist aber auch erkennbar, dass diese Bereiche teilweise überlappen und daher nicht gänzlich trennscharf dargestellt werden können.

In einem exemplarischen Versuch wurde mit den berechneten Merkmalen ein statistischer Klassifizierer, sog. "naiver" Bayes-Klassifizierer trainiert und in einer Kreuzvalidierung evaluiert (vgl. Fig. 20). Hierbei wurde eine Klassifikationsgenauigkeit von 71% erreicht, welche mit den bereits oben dargestellten Überlappungen der Merkmale korrespondiert

Es wurde auch eine Vertauschungsmatrix für einen "naiven" Bayes-Klassifizierer gebildet und dabei eine Klassifikationsgenauigkeit von 71 % erreicht.

Zusammenfassend lösen also obige Ausführungsbeispiele bzw. Konzepte das Problem, eine Quantifizierung von beispielsweise toxischen Auswirkungen von Substanzen auf die beispielsweise *in vitro* Testsysteme zu schaffen. Eine manuelle Sichtprüfung unter dem Mikroskop ist nicht mehr erforderlich und somit auch die damit verbundenen Nachteile des hohen Zeitaufwands, der Subjektivität, der nicht vorhandenen Reproduzierbarkeit und der Möglichkeit des Treffens lediglich qualitativer Aussagen. Vielmehr erlauben obige Ausführungsbeispiele eine zumindest teilautomatische Quantifizierung von beispielsweise toxischen Auswirkungen von Substanzen auf *in vitro* Testsysteme.

Obige Ausführungsbeispiele könne dabei grundsätzlich in etwa folgendem Ablauf folgen.
1. Aufbringen der Substanzen auf das Gewebe *in vitro* und Einwirken für eine definierte Zeit. Optional: Aufbringen einer bekannten Testsubstanz auf einem Teil des Testgewebes zum Ausgleich/Erkennen von Chargen-Variationen (die Testsysteme werden aus Human-Zellen gezüchtet und haben daher biologische Varianz)
2. Präparieren der Proben und Anfertigen von histologischen Schnitten. Diese liegen auf einem Glasobjektträger für die Mikroskopie vor.(Standardverfahren der Pathologie, vgl. z.B. http://de.wikipedia.org/wiki/Histologie)
3. Einscannen des Glasobjektträgers mit einem automatisierten Mikroskop (motorisierter XY-Tisch, z-Achse für Fokussierung, mehrere Objektive, Kamera, Lichtquelle). Der Objektträger wird zuerst in geringer Auflösung (z.B. 1x-Objektiv) mäanderförmig gescannt.
4. Erstellen eines sog. Virtuellen Objektträgers aus den einzelnen aufgenommenen Kamerabildern.
5. Detektion der Gewebeschnitte auf dem Gesamtobjektträger (vgl. beiliegenden Berichtsentwurf Abschnitt 3.2)
6. Einscannen der jeweiligen Gewebeschnitte mit höherer Auflösung (z.B. 40x-Objektiv). Zusammensetzen zu einem Gesamtbild.
7. Eingrenzen des Auswertebereichs von links und rechts (z.B. 10-15% links und rechts abschneiden, nur der Bereich oben an der Epidermis (vgl. Fig. 2). Dieser Schritt kann manuell oder automatisch erfolgen (z.B. indem von der sog. Bounding Box, also dem eingrenzenden Rechteck des Schnittes von links und rechts ein einstellbarer Prozentsatz "abgeschnitten wird).
8. Segmentierung der epidermalen Schicht durch ein automatisches Verfahren (vgl. Fig. 8)
9. Charakterisierung durch Berechnung von quantitativen Merkmalen (vgl. Parameter 1 bis 4)

Es sei erwähnt, dass oben beschriebene Ausführungsbeispiele in den Grundzügigen theoretisch auch auf anderen Arten von Schädigungen anwendbar sind als nur auf toxische. Zusätzlich sind die beschriebenen Ausführungsbeispiele nicht auf künstlich erstellte Gewebeproben begrenzt, sondern allgemein auf alle Hautgewebeschnitte, d.h. gleichermaßen auch auf nativen Hautschnitte oder gar tierische Proben.

Zu dem Schritt der Parameterbestimmung sie noch folgendes erwähnt. Nachdem der Schädigungsgrad auch von der biologischen Varianz der Hautproben abhängen kann, z.B. aufgrund einer unterschiedlichen "Robustheit", ist es sinnvoll von der gleichen Hautprobe auch Samples zu haben, die mit einer bekannten Testsubstanz(en) behandelt werden. Hierdurch kann eine Normierung der Schädigungsgrade erfolgen, z.B. durch Vergleich der erhaltenen Parametern mit den Parametern, die aus einer mit der Testsubstanz(en) behandelten Probe entstanden sind, so dass die Messung letztlich genauer bzw. unabhängiger wird.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar. Einige oder alle der Verfahrensschritte können durch einen Hardware-Apparat (oder unter Verwendung eines Hardware-Apparats), wie zum Beispiel einen Mikroprozessor, einen programmierbaren Computer oder eine elektronische Schaltung ausgeführt werden. Bei einigen Ausführungsbeispielen können einige oder mehrere der wichtigsten Verfahrensschritte durch einen solchen Apparat ausgeführt werden.

Je nach bestimmten Implementierungsanforderungen können Ausführungsbeispiele der Erfindung in Hardware oder in Software implementiert sein. Die Implementierung kann unter Verwendung eines digitalen Speichermediums, beispielsweise einer Floppy-Disk, einer DVD, einer Blu-ray D i s c, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einem programmierbaren Computersystem derart zusammenwirken können oder zusammenwirken, dass das jeweilige Verfahren durchgeführt wird. Deshalb kann das digitale Speichermedium computerlesbar sein.

Manche Ausführungsbeispiele gemäß der Erfindung umfassen also einen Datenträger, der elektronisch lesbare Steuersignale aufweist, die in der Lage sind, mit einem programmierbaren Computersystem derart zusammenzuwirken, dass eines der hierin beschriebenen Verfahren durchgeführt wird.

Allgemein können Ausführungsbeispiele der vorliegenden Erfindung als Computerprogrammprodukt mit einem Programmcode implementiert sein, wobei der Programmcode dahin gehend wirksam ist, eines der Verfahren durchzuführen, wenn das Computerprogrammprodukt auf einem Computer abläuft.

Der Programmcode kann beispielsweise auch auf einem maschinenlesbaren Träger gespeichert sein.

Andere Ausführungsbeispiele umfassen das Computerprogramm zum Durchführen eines der hierin beschriebenen Verfahren, wobei das Computerprogramm auf einem maschinenlesbaren Träger gespeichert ist.

Mit anderen Worten ist ein Ausführungsbeispiel des erfindungsgemäßen Verfahrens somit ein Computerprogramm, das einen Programmcode zum Durchführen eines der hierin beschriebenen Verfahren aufweist, wenn das Computerprogramm auf einem Computer abläuft.

Ein weiteres Ausführungsbeispiel der erfindungsgemäßen Verfahren ist somit ein Datenträger (oder ein digitales Speichermedium oder ein computerlesbares Medium), auf dem das Computerprogramm zum Durchführen eines der hierin beschriebenen Verfahren aufgezeichnet ist.

Ein weiteres Ausführungsbeispiel des erfindungsgemäßen Verfahrens ist somit ein Datenstrom oder eine Sequenz von Signalen, der bzw. die das Computerprogramm zum Durchführen eines der hierin beschriebenen Verfahren darstellt bzw. darstellen. Der Datenstrom oder die Sequenz von Signalen kann bzw. können beispielsweise dahin gehend konfiguriert sein, über eine Datenkommunikationsverbindung, beispielsweise über das Internet, transferiert zu werden.

Ein weiteres Ausführungsbeispiel umfasst eine Verarbeitungseinrichtung, beispielsweise einen Computer oder ein programmierbares Logikbauelement, die dahin gehend konfiguriert oder angepasst ist, eines der hierin beschriebenen Verfahren durchzuführen.

Ein weiteres Ausführungsbeispiel umfasst einen Computer, auf dem das Computerprogramm zum Durchführen eines der hierin beschriebenen Verfahren installiert ist.

Bei manchen Ausführungsbeispielen kann ein programmierbares Logikbauelement (beispielsweise ein feldprogrammierbares Gatterarray, ein FPGA) dazu verwendet werden, manche oder alle Funktionalitäten der hierin beschriebenen Verfahren durchzuführen. Bei manchen Ausführungsbeispielen kann ein feldprogrammierbares Gatterarray mit einem Mikroprozessor zusammenwirken, um eines der hierin beschriebenen Verfahren durchzuführen. Allgemein werden die Verfahren bei einigen Ausführungsbeispielen seitens einer beliebigen Hardwarevorrichtung durchgeführt. Diese kann eine universell einsetzbare Hardware wie ein Computerprozessor (CPU) sein oder für das Verfahren spezifische Hardware, wie beispielsweise ein ASIC.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

### Literatur

[1] Falkenberg F.W., Weichert H., Krane24 M., Bartels I., Nagels H.O., Behn I., Fiebig H. (1995) In-vitro-Produktion von monoklonalen Antikörpern in hohen Konzentrationen in neuen und einfach bedienbaren Modular-Minifermenter. In: Forschung ohne Tierversuche, Schöffl H. et al (Editor), Springer-Verlag) Wien-New York.
[2] Schade R., Pfister C., Halatsch R., Henklein P. (1991). Polyclonal igY antibodies from chicken egg yolk-an alternative to the production of mammlian igG type antibodies in Rabbits, ATLA 19, 403-419.
[3] Otsu N. A threshold selection method from grey level histograms. IEEE Transactions on Systems, Man, and Cybernetics. New York 9.1979, S.62-66.
[4] Perona P., Malik J. (1990). Scale-space and edge detection using anisotropic diffusion. IEEE Transactions on Pattern Analysis and Machine Intelligence, 12 (7): 629-639.
[5] Bezdek J.C. Pattern Recognition with Fuzzy Objective Function Algorithms. Plenum Press, New York 1981

## Patentansprüche

1. Vorrichtung zur Quantifizierung einer Schädigung eines Hautgewebeschnittes anhand eines Farbbildes des Hautgewebeschnittes, mit:
einer Einrichtung (92) zum Konvertieren des Farbbildes (92) in ein Grauwertbild (94);
eine Einrichtung (104) zum Zuweisen von Bildpunkten des Grauwertbildes zu einem jeweiligen Label aus Hintergrund, Epidermis oder Dermis unter Verwendung eines Bildverarbeitungsalgorithmus, um ein Labelbild zu erhalten;
eine Einrichtung (108) zum, entlang einer Richtung, die von einer der Dermis abgewandten Seite auf die Epidermis des Hautgewebeschnittes weist, Bestimmen eines Außeroberflächenrands (116) der Epidermis und Anwenden eines Füllalgorithmus von dem Außenoberflächenrand (116) aus auf das Labelbild, um einen mit dem Außeroberflächenrand (116) zusammenhängenden und sich von dem Außeroberflächenrand (116) in der Richtung zur Dermis hin erstreckenden Epidermisbereich (118) zu bestimmen;
einer Einrichtung (130) zum Lokalisieren der Epidermis in dem Labelbild unter Verwendung des Epidermisbereichs; und
einer Einrichtung zum Bestimmen von Parametern, die charakteristische Eigenschaften einer Form der lokalisierten Epidermis beschreiben.

2. Vorrichtung gemäß Anspruch 1, bei der die Einrichtung zum Konvertieren ausgebildet ist, die Konvertierung durch gewichtete Summation von Farbkanälen des Farbbildes durchzuführen.

3. Vorrichtung gemäß einem der Ansprüche 1 oder 2, bei der die Einrichtung zum Zuweisen eines Labels so ausgebildet ist, dass der Bildverarbeitungsalgorithmus einem Fuzzy-Cluster oder K-Means Algorithmus entspricht.

4. Vorrichtung gemäß einem der vorhergehenden Ansprüche, bei der die Einrichtung zum Bestimmen und Anwenden ausgebildet ist, um einen morphologischen Operator zu verwenden, um Epidermis-Inseln und/oder Epidermis-Schnittreste auf einer der Dermis abgewandten Seite der Epidermis bei der Bestimmung des Außeroberflächenrands (116) auszublenden, so dass der Außeroberflächenrand näher zur Dermis verläuft.

5. Vorrichtung gemäß einem der vorhergehenden Ansprüche, bei der die Einrichtung zum Bestimmen und Anwenden ausgebildet ist, ferner einen Skelettierungsalgorithmus auf den Epidermisbereich anzuwenden, um Auswüchse (128) des Epidermisbereichs, die von einem sich entlang einer Längserstreckungsrichtung (124) des Epidermisbereichs (118) erstreckenden Skelettierungsergebnis des Skelettierungsalgorithmus weiter als ein vorbestimmtes Maß entfernt liegen, zu entfernen.

6. Vorrichtung gemäß einem der vorhergehenden Ansprüche, bei der die Einrichtung zum Lokalisieren ausgebildet ist, um zur Lokalisierung der Epidermis Bildpunkte des Labelbildes als zur Epidermis gehörig auszuwählen, die in dem Epidermisbereich liegen.

7. Vorrichtung gemäß einem der vorhergehenden Ansprüche, bei der die Einrichtung zum Bestimmen der Parameter ausgebildet ist, um zumindest einen der folgenden Parameter zu berechnen:
a) eine Ausdehnung der lokalisierten Epidermis in der von der der Dermis abgewandten Seite auf die Epidermis des Hautgewebeschnittes weisenden Richtung für unterschiedliche Positionen entlang einer Längserstreckungsrichtung der Epidermis;
b) eine Länge einer der Dermis abgewandten Epidermiskante der lokalisierten Epidermis, soweit die Epidermis innerhalb eines Kreises mit einem vorbestimmten Radius um eine jeweilige Position entlang der Epidermiskante liegt, für unterschiedliche Positionen entlang der Epidermiskante;
c) eine Anzahl von Überschneidungen einer der Dermis abgewandten Epidermiskante der lokalisierten Epidermis mit einem Kreis mit dem vorbestimmten Radius um eine jeweilige Position entlang der Epidermiskante für unterschiedliche Positionen entlang der Epidermiskante;
d) ein Histogramm einer Verteilung einer Länge einer der Dermis abgewandten Epidermiskante der lokalisierten Epidermis, soweit die Epidermis innerhalb eines Kreises mit einem vorbestimmten Radius um eine jeweilige Position entlang der Epidermiskante liegt, für Positionen entlang der Epidermiskante; und
e) eine Anzahl von Ästen einer Skelettierung der lokalisierten Epidermis in der Nähe der Epidermiskante.

8. Vorrichtung gemäß einem der vorhergehenden Ansprüche, bei der die Vorrichtung ferner eine Einrichtung zum Bestimmen eines Maßes für die Schädigung auf Basis der Parameter aufweist.

9. Vorrichtung gemäß Anspruch 8, bei der die Vorrichtung ausgebildet ist, die Parameter auch für eine mit einer bekannten Testsubstanz behandelten Hautgewebeschnitt zu bestimmen, wobei die Einrichtung zum Bestimmen eines Maßes für die Schädigung ausgebildet ist, das Maß auf Basis eines Vergleichs der Parameter des Hautgewebeschnitts mit den Parametern des mit der bekannten Testsubstanz behandelten Hautgewebeschnitts zu bestimmen.

10. Vorrichtung gemäß einem der vorhergehenden Ansprüche, bei der die Vorrichtung ferner eine Einrichtung (30) zum Aufnehmen des Farbbildes aufweist.

11. Vorrichtung gemäß einem der vorhergehenden Ansprüche, bei der die Vorrichtung ferner ein Mikroskop mit einem Objektträgerverfahrgerät (34) aufweist, die ausgebildet sind, um so zusammenzuwirken, dass sich überlappende Einzelbilder des Hautgewebeschnittes erzeugt werden, sowie eine Einrichtung (36) zum Aneinanderfügen der Einzelbilder, um das Farbbild zu erhalten.

12. Vorrichtung gemäß einem der vorhergehenden Ansprüche, bei der die Vorrichtung ferner ein Mikroskop (32) mit einer größeren und einer feineren Vergrößerung, ein Objektträgerverfahrgerät (34) und eine Steuereinrichtung (40) aufweist, die ausgebildet ist, um das Mikroskop (32) und das Objektträgerverfahrgerät (34) so anzusteuern, dass zunächst eine Übersichtsaufnahme mit der groben Vergrößerung erzeugt wird, in welcher die Steuereinrichtung (40) eine Position des Hautgewebeschnittes auf einem Objektträger (42) unter Auswertung einer bimodalen Verteilung von Grauwerten in dem Übersichtsbild bestimmt, und der Hautgewebeschnitt mit der feinen Vergrößerung an der bestimmten Position aufgenommen wird, um das Farbbild des Hautgewebeschnittes zu erhalten.

13. Verfahren zur Quantifizierung einer Schädigung eines Hautgewebeschnittes anhand eines Farbbildes des Hautgewebeschnittes, mit:
Konvertieren des Farbbildes in ein Grauwertbild;
Zuweisen von Bildpunkten des Grauwertbildes zu einem jeweiligen aus Hintergrund, Epidermis oder Dermis unter Verwendung eines Bildverarbeitungsalgorithmus, um ein Labelbild zu erhalten;
entlang einer Richtung, die von einer der Dermis abgewandten Seite auf die Epidermis des Hautgewebeschnittes weist, Bestimmen eines Außeroberflächenrands der Epidermis und Anwenden eines Füllalgorithmus von dem Außenoberflächenrand aus auf das Labelbild, um einen mit dem Außeroberflächenrand zusammenhängenden und sich von dem Außeroberflächenrand in der Richtung zur Dermis hin erstreckenden Epidermisbereich zu bestimmen;
Lokalisieren der Epidermis in dem Labelbild unter Verwendung des Epidermisbereichs; und
Bestimmen von Parametern, die charakteristische Eigenschaften einer Form der lokalisierten Epidermis beschreiben.

14. Verfahren gemäß Anspruch 13., wobei das Verfahren ferner ein histologisches Schneiden einer Hautgewebeprobe aufweist, um den Hautgewebeschnitt zu erhalten.

15. Verfahren gemäß Anspruch 13 oder 14, das ferner einen Einwirkschritt (20) aufweist, bei dem der Hautgewebeschnitt oder das Hautgewebe, aus der der Hautgewebeschnitt per histologischem Schnitt erhalten wird, einem zerstörenden Einfluss Substanz ausgesetzt wird.

16. Computerprogramm mit einem Programmcode zur Durchführung des Verfahrens nach Ansprüche 13, wenn das Programm auf einem Computer abläuft.
